# EUROPEAN PATENT SPECIFICATION

(11) **EP 4 037 660 B1**
(45) Date of publication and mention of the grant of the patent: **22.11.2023**
(21) Application number: 20789268.8
(22) Date of filing: 28.09.2020
(51) Int. Cl.: A61K 9/00, A61K 47/34, A61K 38/09, A61K 38/29, A61K 38/31, C08G 63/00, C08G 64/00

(54) **LIQUID POLYMER COMPOSITIONS AND SYSTEMS FOR EXTENDED DELIVERY OF PEPTIDES AS ACTIVE PHARMACEUTICAL INGREDIENTS**
FLÜSSIGE POLYMERZUSAMMENSETZUNGEN UND SYSTEME ZUR VERLÄNGERTEN ABGABE VON PEPTIDEN ALS PHARMAZEUTISCHE WIRKSTOFFE
COMPOSITIONS POLYMÈRES LIQUIDES ET SYSTÈMES D'ADMINISTRATION PROLONGÉE DE PEPTIDES EN TANT QU'INGRÉDIENTS PHARMACEUTIQUES ACTIFS

(30) Priority: 30.09.2019 US 201962908340 P
(43) Date of publication of application: 10.08.2022
(73) Proprietor: Tolmar International Limited, Dublin 2, DO2 T380 (IE)
(72) Inventor: GOTTAM, Hima Bindu, Fort Collins, Colorado 80536 (US); KUMMEROW CASAS, Gerhard, Guilford, Connecticut 06437 (US); MIDDLETON, John Charles, Fort Collins, Colorado 80525 (US); NANGIA, Avinash, Fort Collins, Colorado 80528 (US)
(74) Representative: Steffan & Kiehne Patentanwälte PartG mbB
(86) International application number: PCT/IB2020/059058
(87) International publication number: WO 2021/064550

(56) References cited:
- EP-A1- 2 859 887
- WO-A2-2007/019439
- US-A1- 2007 265 356
- US-A1- 2017 066 874

## Description

### FIELD OF THE INVENTION

This application pertains to the field of biodegradable liquid polymer compositions that may be administered into the body with syringes or needles and that may be utilized to deliver a drug, such as a peptide, into the body over an extended period of time.

### BACKGROUND OF THE INVENTION

Biodegradable polymers are well known for their use in biomedical applications, such as sutures, surgical clips, staples, implants, and drug delivery systems. Such polymers include polyglycolides, polylactides, polycaprolactones, polyanhydrides, polyorthoesters, polydioxanones, polyacetals, polyesteramides, polyamides, polyurethanes, polycarbonates, poly(amino acids), polyphosphazenes, polyketals, polyphosphate esters, polyhydroxybutyrates, polyhydroxyalerates, and polyalkylene oxalates.

Initially, the biodegradable polymers were solid materials that were used to form solid articles such as sutures, staples, surgical clips, tissue scaffolds, implants or microcapsules and microparticles. Because the polymers were solids, all of their applications in the biomedical field required that the polymeric structures be formed outside the body, and then inserted into the body for their use.

U.S. Patent 5,278,201 to Dunn et al. (the "201 patent") overcame the administration problems with the solid implants by dissolving the solid biodegradable polymers in a biocompatible solvent and injecting the solution into the body using standard syringes and needles where the polymer in the solution precipitates or coagulates upon contact with aqueous body fluid to form a solid implant matrix. The delivery system described in the `201 patent offered a number of advantages, including the ease of manufacture of the polymer solution, the incorporation of the drug into the polymer solution just prior to administration leading to increased drug and polymer stability as well as no loss of drug during the manufacturing process, and the ability to terminally sterilize the polymer solution as well as the drug. However, there remained several disadvantages with this *in situ* forming polymeric system. Because the polymers used were solids with relatively high molecular weights, the polymer solutions formed from the combination of the solid polymers and the biocompatible solvents were quite viscous. Because of the high viscosity, large bore 18-21 gauge needles were required for administration and considerable injection force was needed. In addition, the viscous solutions were not easily injected into muscle tissue and the solid implants formed from these polymer solutions tended to cause local irritation of the muscular tissue. For this reason, the foregoing polymer solutions were normally injected subcutaneously where the material would form quite distinct and noticeable bumps.

U.S. Patent 8,187,640 to Dunn (the "'640 patent") addressed problems associated with the solid implants of the `201 patent. The '640 patent disclosed compositions of a biodegradable liquid polymer combined with a biocompatible solvent, which solvent would dissipate when the liquid polymer/solvent compositions were placed in a body, thereby forming a viscous liquid polymer material in the form of a film, a coating, a plug or other mass. The viscous liquid polymer material does not solidify upon injection into the body, but rather remains *in situ* in a viscous liquid form and, when combined with a drug, provides both an initial burst and extended release of the drug.

PCT Publication No. WO2017024027 describes the making of the low viscosity liquid polymeric delivery system as disclosed in the '640 patent to determine the rate and duration of the release of drugs following subcutaneous administration of the drug-loaded delivery system. It was determined that the delivery system of the '640 patent is not suitable for long-term extended delivery of drugs beyond, *e.g.*, 14 days. PCT Publication No. WO2017024027 discloses a different liquid polymer composition than that described in the '640 patent, which provided a markedly improved extended release of drugs as compared to the '640 patent. The liquid polymer composition described in PCT Publication No. WO2017024027 included a biodegradable liquid polymer with at least one carboxylic acid end group, and a ratio of monomer units to carboxylic acid end groups between about 5:1 and about 90:1.

Maintaining the chemical and physical stability of the active pharmaceutical ingredient throughout the lifecycle of the pharmaceutical product is a significant challenge in the development of polymeric pharmaceutical delivery vehicles whose active ingredients are susceptible to acid degradation, such as peptide drugs. For example, during hydrolytic degradation of an *in vivo* polymer implant, the pH of the environment surrounding the polymer implant decreases, which can result in peptide instability. Previous studies have also shown that peptide drugs are prone to degradation in the presence of lactide- and/or glycolide-containing polymers, depending on the primary sequence and higher-order structure of the peptide. There is thus a need in the art for polymeric pharmaceutical delivery compositions and systems comprising peptide drugs, in which the peptide drug remains stable to provide a suitable extended release for a target application.

### SUMMARY OF THE INVENTION

It is one aspect of the present invention to provide a pharmaceutical composition, comprising an active pharmaceutical ingredient comprising a peptide or a pharmaceutically acceptable ester or salt thereof, wherein the peptide comprises at least one accessible amine group; and a biodegradable liquid block copolymer comprising a copolymer block comprising: (1) monomer residues selected from the group consisting of D,L-lactide, D-lactide, L-lactide, and glycolide, and combinations thereof and (2) monomer residues selected from the group consisting of ε-caprolactone, trimethylene carbonate, and combinations thereof; and a polymer block comprising a low-molecular weight polyethylene glycol (PEG); wherein the biodegradable liquid block copolymer is synthesized by initiation with the low-molecular weight PEG; and wherein a viscosity of the biodegradable liquid block copolymer does not spontaneously increase with an increase in temperature.

The biodegradable liquid block copolymer is not a reverse thermal gel.

The end groups of the biodegradable liquid block copolymer are not covalently modified.

In embodiments, the biodegradable block copolymer may be a tri-block copolymer according to the formula: A-B-A, wherein A is the copolymer block of (i), and B is the polymer block comprising PEG.

In embodiments, the biodegradable block copolymer may be a di-block copolymer according to the formula: A-B or B-A, wherein A is the copolymer block of (i) and B is the polymer block comprising PEG, and the PEG is methoxy-PEG.

In embodiments, the copolymer block of (i) may have a molar ratio of lactide or glycolide monomer residues to caprolactone and/or trimethylene carbonate monomer residues between about 10:90 and about 90:10.

In embodiments, the copolymer block of (i) may have a molar ratio of lactide or glycolide monomer residues to caprolactone and/or trimethylene carbonate monomer residues between about 20:80 and about 80:20.

In embodiments, the copolymer block of (i) may have a molar ratio of lactide or glycolide monomer residues to caprolactone and/or trimethylene carbonate monomer residues between about 25:75 and about 75:25.

In embodiments, the copolymer block of (i) may have a molar ratio of lactide monomer residues to caprolactone and/or trimethylene carbonate monomer residues of 75:25.

In embodiments, a molar ratio of ethylene glycol residues in the low-molecular weight PEG to all other monomer residues in the biodegradable copolymer may be between about 10:90 and 50:50.

In embodiments, a molar ratio of ethylene glycol residues in the low-molecular weight PEG to all other monomer residues in the biodegradable copolymer may be at least about 10:90.

In embodiments, a molar ratio of ethylene glycol residues in the low-molecular weight PEG to all other monomer residues in the biodegradable copolymer may be at least about 20:80.

In embodiments, a molar ratio of ethylene glycol residues in the low-molecular weight PEG to all other monomer residues in the biodegradable copolymer may be at least about 30:70.

In embodiments, a molar ratio of lactide or glycolide monomer residues to caprolactone and/or trimethylene carbonate monomers to ethylene glycol residues in the biodegradable copolymer may be X:Y:Z, where X can be any number between about 25 and about 75, Y can be any number between about 5 and about 45, and Z can be any number between about 5 and about 55, such that the sum of X, Y, and Z is 100.

In embodiments, the polyethylene glycol may have a number average molecular weight of 200 to 2000 daltons.

In embodiments, a number average molecular weight of the low-molecular weight polyethylene glycol may be less than or about 900 daltons.

In embodiments, the number average molecular weight of the low-molecular weight polyethylene glycol may be less than or about 600 daltons.

In embodiments, the number average molecular weight of the low-molecular weight polyethylene glycol may be less than or about 400 daltons.

In embodiments, the number average molecular weight of the low-molecular weight polyethylene glycol is less than or about 300 daltons.

In embodiments, the pharmaceutical composition may further comprise a biocompatible solvent.

In embodiments, the pharmaceutical composition may further comprise a divalent cation.

In embodiments, the divalent cation may be selected from the group consisting of magnesium, calcium, and zinc.

In embodiments, the divalent cation may be provided as a metal salt. The metal salt may, but need not, be selected from the group consisting of magnesium acetate, magnesium chloride, calcium chloride, zinc acetate, and zinc chloride.

In embodiments, the metal salt may be magnesium acetate, and the magnesium acetate may be at a concentration of between about 0.01 mg/mL and about 2.75 mg/mL of the pharmaceutical composition.

In embodiments, the metal salt may be magnesium chloride, and the magnesium chloride may be at a concentration of between about 0.01 mg/mL and about 3.75 mg/mL of the pharmaceutical composition.

In embodiments, the metal salt may be calcium chloride, and the calcium chloride may be at a concentration of between about 0.01 mg/mL and about 1.6 mg/mL of the pharmaceutical composition.

In embodiments, the metal salt may be zinc acetate, and the zinc acetate may be at a concentration of between about 0.01 mg/mL and about 8.2 mg/mL of the pharmaceutical composition.

In embodiments, the metal salt may be zinc chloride, and the zinc chloride may be at a concentration of between about 0.01 mg/mL and about 1.4 mg/mL of the pharmaceutical composition.

In embodiments, the peptide may comprise at least two basic amino acids selected from the group consisting of arginine, histidine, lysine, and combinations thereof.

It is another aspect of the present disclosure to provide a pharmaceutical composition, comprising an active pharmaceutical ingredient comprising a linear peptide or a pharmaceutically acceptable ester or salt thereof, wherein the linear peptide does not have an accessible amine group; a biocompatible solvent; a biodegradable copolymer comprising lactide residues and monomer residues selected from the group consisting of caprolactone, trimethylene carbonate, and combinations thereof, wherein the biodegradable copolymer comprises at least one carboxylic acid end group; and a divalent cation.

In embodiments, the divalent cation may be selected from the group consisting of magnesium and zinc.

In embodiments, the divalent cation may be provided as a metal salt. The metal salt may, but need not, be selected from the group consisting of magnesium acetate, zinc chloride, and zinc acetate.

In embodiments, the metal salt may be magnesium acetate, and the magnesium acetate may be at a concentration of between about 0.01 mg/mL and about 1.65 mg/mL of the pharmaceutical composition.

In embodiments, the metal salt may be zinc chloride, and the zinc chloride may be at a concentration of between about 0.01 mg/mL and about 1.1 mg/mL of the pharmaceutical composition.

In embodiments, the metal salt may be zinc acetate, and the zinc acetate may be at a concentration of between about 0.01 mg/mL of the pharmaceutical composition and about 7.15 mg/mL of the pharmaceutical composition.

In embodiments, the biodegradable copolymer may be synthesized with a hydroxy acid initiator. The hydroxy acid initiator may, but need not, be glycolic acid.

It is another aspect of the present disclosure to provide a pharmaceutical composition, comprising an active pharmaceutical ingredient comprising a cyclic peptide or a pharmaceutically acceptable ester or salt thereof, wherein the cyclic peptide has no accessible amine groups or one accessible amine group; a biocompatible solvent; a biodegradable copolymer comprising lactide residues and monomer residues selected from the group consisting of caprolactone, trimethylene carbonate, and combinations thereof, wherein the biodegradable copolymer comprises at least one carboxylic acid end group; and a divalent cation.

In embodiments, the divalent cation may be zinc.

In embodiments, the divalent cation may be provided as a metal salt. The metal salt may, but need not, be selected from the group consisting of zinc chloride and zinc acetate.

In embodiments, the metal salt may be zinc chloride, and the zinc chloride may be at a concentration of between about 0.01 mg/mL and about 1.2 mg/mL of the pharmaceutical composition.

In embodiments, the metal salt may be zinc acetate, and the zinc acetate may be at a concentration of between about 0.01 mg/mL and about 7.3 mg/mL of the pharmaceutical composition.

In embodiments, the biodegradable copolymer may be formed with a hydroxy acid initiator. The hydroxy acid initiator may, but need not, be glycolic acid.

In embodiments, a weight-average molecular weight of the biodegradable copolymer may be between about 1 kDa and about 35 kDa.

In embodiments, the biocompatible solvent may comprise at least one selected from the group consisting of N-methyl-2-pyrrolidone (NMP), acetone, butyrolactone, ε-caprolactone, N-cycylohexyl-2-pyrrolidone, diethylene glycol monomethyl ether, dimethyl acetamide, dimethyl formamide, dimethyl sulfoxide (DMSO), ethyl acetate, ethyl lactate, N-ethyl-2-pyrrolidone, glycerol formal, glycofurol, N-hydroxyethyl-2-pyrrolidone, isopropylidene glycerol, lactic acid, methoxypolyethylene glycol, methoxypropylene glycol, methyl acetate, methyl ethyl ketone, methyl lactate, low-molecular weight (MW) polyethylene glycol (PEG), polysorbate 80, polysorbate 60, polysorbate 40, polysorbate 20, polyoxyl 35 hydrogenated castor oil, polyoxyl 40 hydrogenated castor oil, sorbitan monolaurate, sorbitan monostearate, sorbitan monooleate, benzyl alcohol, n-propanol, isopropanol, tert-butanol, propylene glycol, 2-pyrrolidone, α-tocopherol, triacetin, tributyl citrate, acetyl tributyl citrate, acetyl triethyl citrate, triethyl citrate, esters thereof, and combinations and mixtures thereof.

In embodiments, the biocompatible solvent may comprise N-methyl-2-pyrrolidone (NMP).

In embodiments, the composition may have a viscosity at room temperature suitable for injection.

In embodiments, after administration of the pharmaceutical composition to an animal, the biocompatible solvent may dissipate in the body of the animal and the biodegradable copolymer may form a biodegradable, non-solid implant in situ in the body of the animal.

It is another aspect of the present invention to use a pharmaceutical composition as herein described as a medicament or in the treatment of a disease.

It is another aspect of the present disclosure to provide a method of treating a subject with an active pharmaceutical ingredient, comprising administering to the subject a pharmaceutical composition as herein described.

It is another aspect of the present disclosure to provide a delivery system for administration of a pharmaceutical composition, comprising a syringe; and a pharmaceutical composition as herein described, wherein the composition is contained within the syringe.

In embodiments, the syringe may be a mixing syringe.

In embodiments, the syringe may be an autoinjector.

### BRIEF DESCRIPTION OF THE DRAWINGS

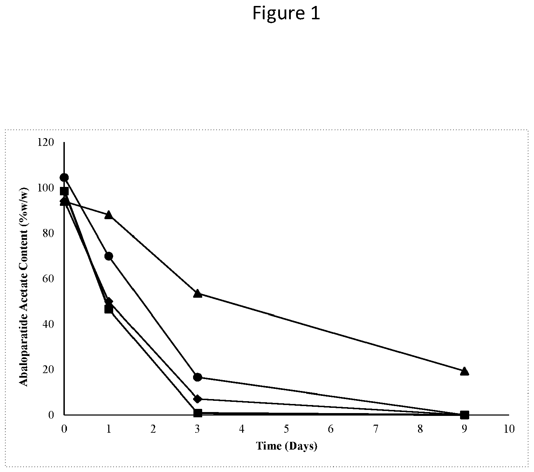

Fig. 1 shows the percent recovery of abaloparatide acetate (AA) as a function of time (days) at 25 °C for LPT formulations comprising a lactide:caprolactone copolymer initiated with different polymer initiators: acid initiated polymer with a lactide-to-caprolactone molar ratio of about 25:75 (◆, AAX1), acid initiated polymer with a lactide-to-caprolactone molar ratio of about 75:25 (■, AAX2), PEG-300 initiated polymer with a lactide-to-caprolactone molar ratio of about 75:25 (▲, AAX3), and dodecanol initiated polymer with a lactide-to-caprolactone molar ratio of about 75:25 (•, AAX4).

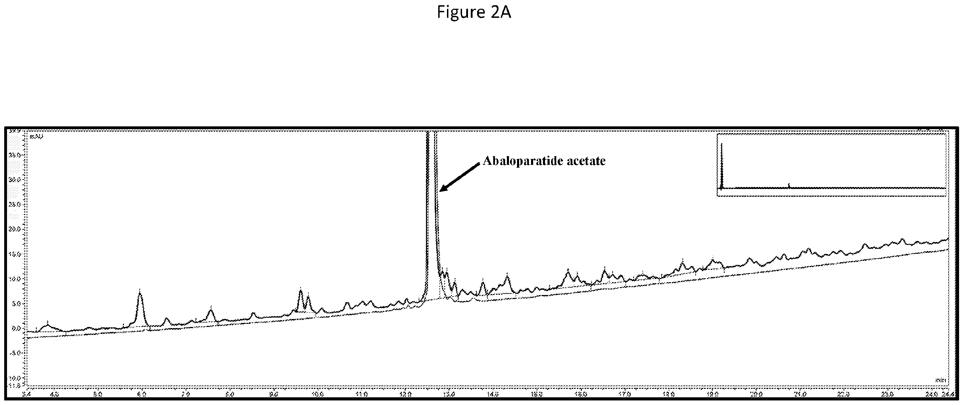

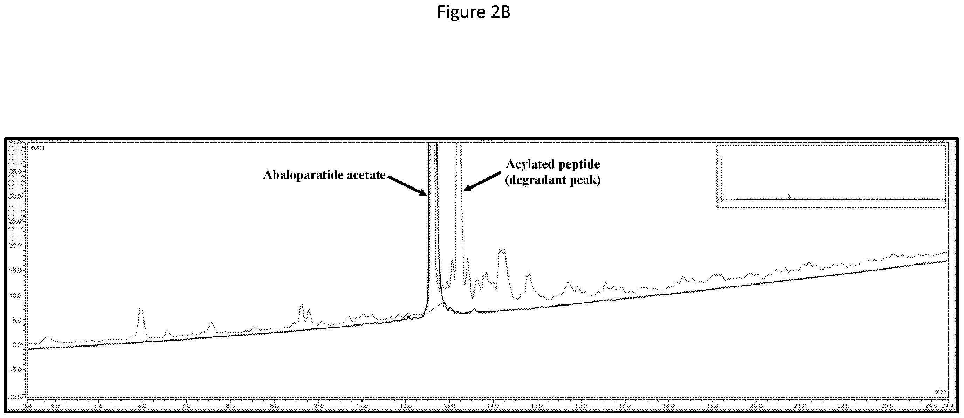

Figs. 2A and 2B show the HPLC chromatograms of a LPT formulation comprising an acid-initiated LPT-polymer and abaloparatide acetate (AA). Fig. 2A is a chromatogram of a fresh sample of the formulation (0 days) and Fig 2B is a chromatogram of a sample stored for 2 days at 25 °C.

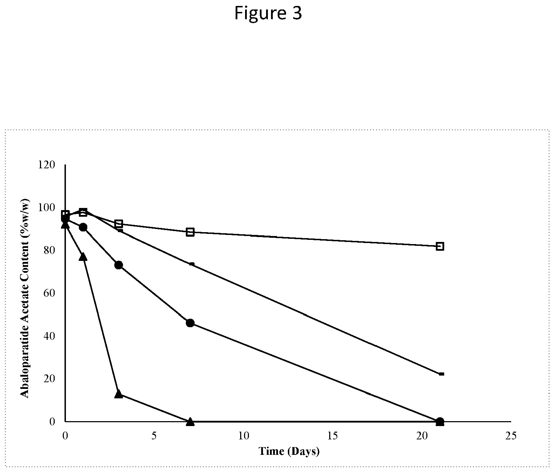

Fig. 3 shows the percent recovery of abaloparatide acetate (AA) as a function of time (days) at 37 °C for LPT formulations comprising a PEG-300 initiated lactide:caprolactone (75:25) copolymer in the presence of divalent cation salts: magnesium chloride (□, AA1), calcium chloride (■, AA2), zinc chloride (•, AA3), and the control formulation containing no salt (▲, AAX8).

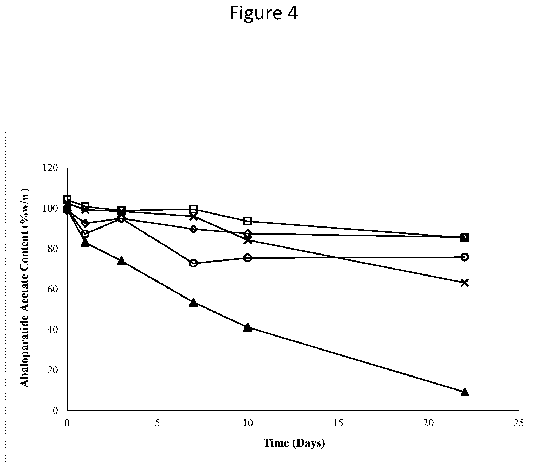

Fig. 4 shows the percent recovery of abaloparatide acetate (AA) as a function of time (days) at 37 °C for LPT formulations comprising PEG-300 initiated lactide:caprolactone copolymer in the presence of magnesium salts: 1.1 mg/mL ("low" concentration) magnesium acetate (∘, AA4), 2.2 mg/mL ("high" concentration) magnesium acetate (0, AA5), 1.25 mg/mL "low" concentration of magnesium chloride (x, AA6), 2.5 mg/mL "high" concentration of magnesium chloride (□, AA7), and control formulation containing no salt (▲, AAX9).

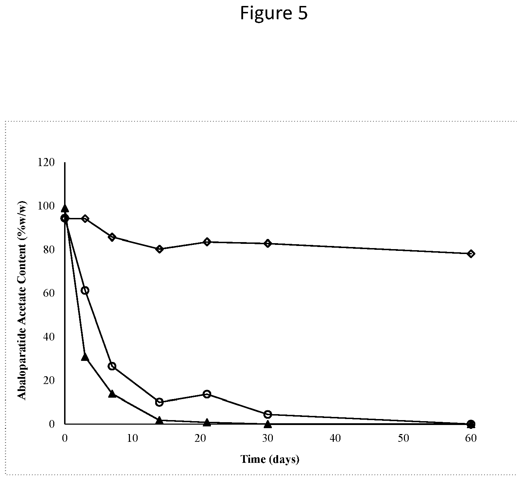

Fig. 5 shows the percent recovery of abaloparatide acetate (AA) as a function of time (days) at 37 °C for LPT formulations comprising a PEG-300 initiated lactide:caprolactone copolymer (75:25) in the presence of magnesium acetate: 0.55 mg/mL ("low" concentration) of magnesium acetate (∘, AA8), 1.1 mg/mL ("high" concentration) of magnesium acetate (0, AA9), and control formulation containing no salt (▲, AAX10).

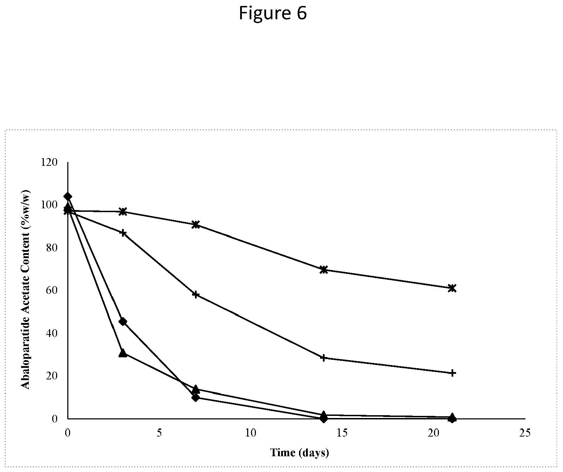

Fig. 6 shows the percent recovery of abaloparatide acetate (AA) as a function of time (days) at 37 °C for LPT formulations comprising a PEG-300 initiated lactide:caprolactone copolymer (75:25) in the presence of zinc salts: 2.5 mg/mL ("low" concentration) of zinc acetate ( 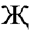 , AA10), 5.68 mg/mL ("high" concentration) of zinc acetate (+, AA11), 1.0 mg/mL of zinc chloride (◆, AA12), and control formulation containing no salt (▲, AAX11).

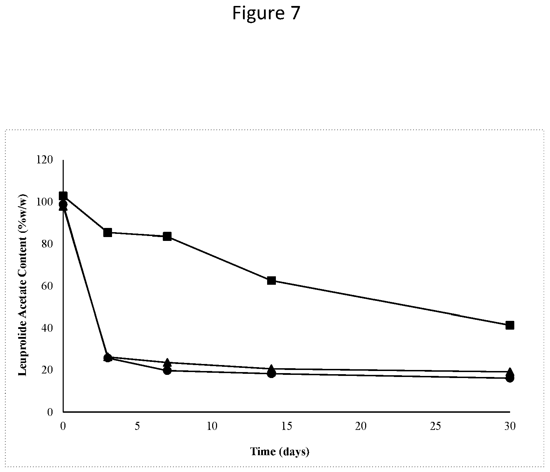

Fig. 7 shows the percent recovery of leuprolide acetate (LA) released as a function of time (days) at 37 °C for LPT formulations comprising a lactide:caprolactone copolymer (75:25) initiated with different polymer initiators: acid initiated polymer (■, LAX1), PEG-300 initiated polymer (▲, LAX2), and dodecanol initiated polymer (•, LAX3).

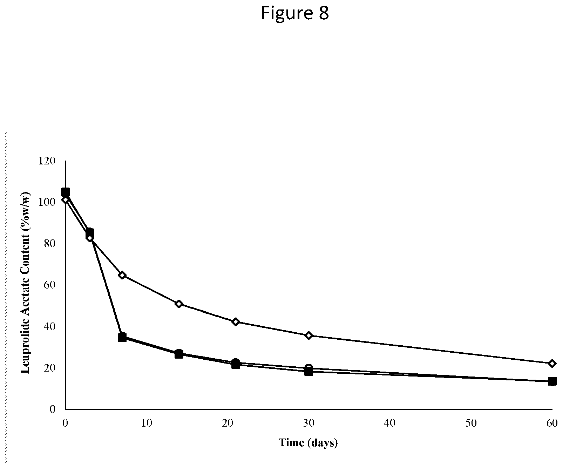

Fig. 8 shows the percent recovery of leuprolide acetate (LA) as a function of time (days) at 37 °C for LPT formulations comprising an acid initiated lactide:caprolactone copolymer (75:25) in the presence of magnesium acetate: 0.53 mg/mL ("low" concentration) of magnesium acetate (∘, LA1), 1.08 mg/mL ("high" concentration) of magnesium acetate (0, LA2), and control formulation containing no salt (■, LAX4).

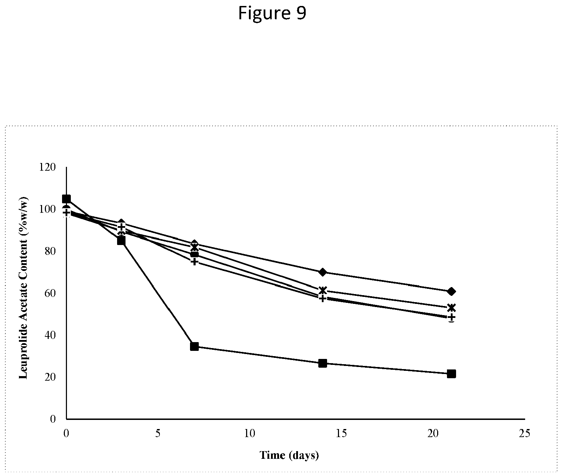

Fig. 9 shows the percent recovery of leuprolide acetate (LA) as a function of time (days) at 37 °C for LPT formulations comprising an acid initiated lactide:caprolactone copolymer (75:25) in the presence of zinc salts: 2.14 mg/mL ("low" concentration) of zinc acetate ( 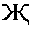 , LA3), 5.0 mg/mL ("high" concentration) of zinc acetate (+, LA4), 0.15 mg/ML ("low" concentration) of zinc chloride (•, LA5), 0.93 mg/mL ("high" concentration) of zinc chloride (◆, LA6), and control formulation containing no salt (■, LAX4).

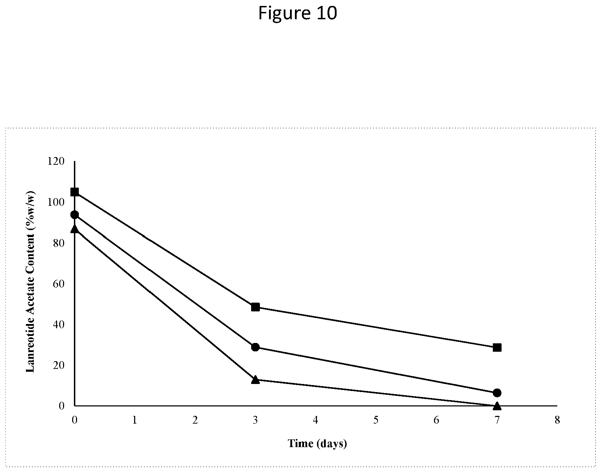

Fig. 10 shows the percent recovery of lanreotide acetate (LNA) as a function time (days) at 37 °C for LPT formulations comprising a lactide:caprolactone copolymer (75:25) initiated with different polymer initiators: acid initiated polymer (■, LNAX1), dodecanol initiated polymer (•, LNAX2), and PEG-300 initiated polymer (▲, LNAX3).

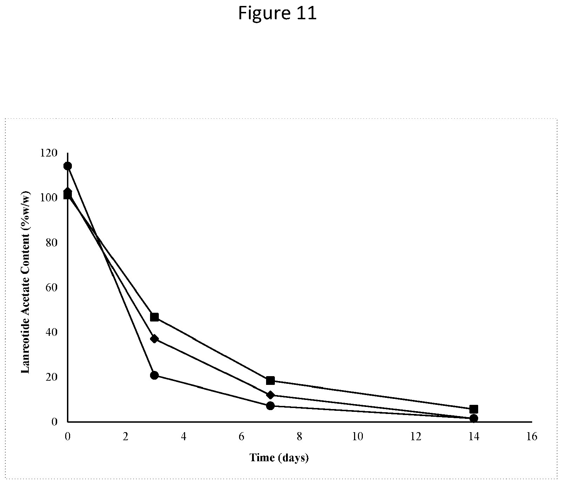

Fig. 11 shows the percent recovery of lanreotide acetate (LNA) as a function of time (days) at 37 °C for LPT formulations comprising an acid-initiated lactide:caprolactone copolymer (75:25) in the presence of magnesium acetate: 0.55 mg/mL ("low" concentration) of magnesium acetate (•, LNA1), 1.1 mg/mL ("high" concentration) of magnesium acetate (◆, LNA2), and control formulation containing no salt (■, LNAX4).

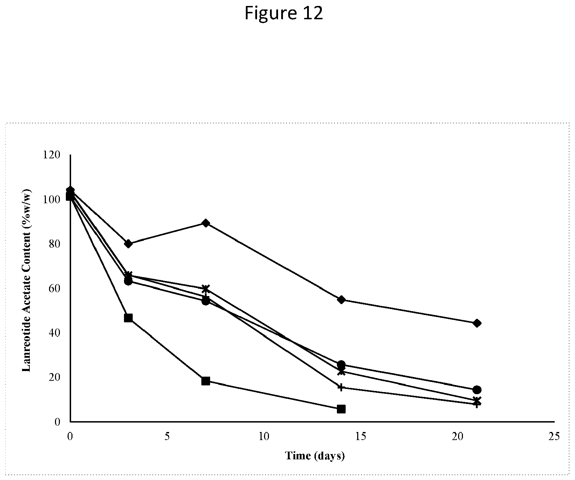

Fig. 12 shows the percent recovery of lanreotide acetate (LNA) as a function of time (days) at 37 °C for LPT formulations comprising an acid-initiated lactide:caprolactone copolymer (75:25) in the presence of zinc salts: 2.3 mg/mL ("low" concentration) of zinc acetate ( 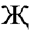 , LNA3), 5.0 mg/mL ("high" concentration) of zinc acetate (+, LNA4), 0.2 mg/mL ("low" concentration) of zinc chloride (•, LNA5), 1.0 mg/mL ("high" concentration) of zinc chloride (◆, LNA6), and control formulation containing no salt (■, LNAX4).

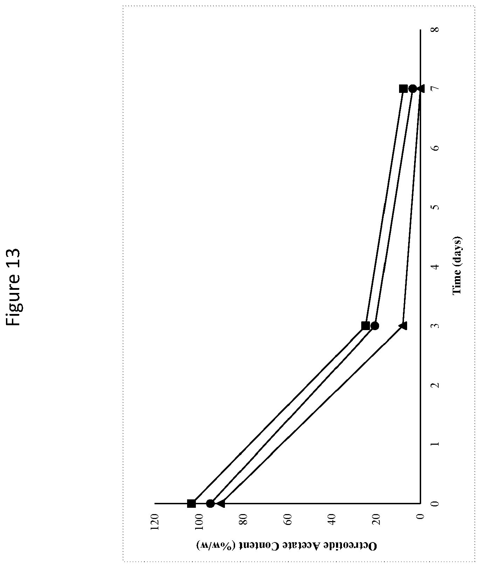

Fig. 13 shows the percent recovery of octreotide acetate (OA) as a function of time (days) at 37 °C for LPT formulations comprising a lactide:caprolactone copolymer (75:25) initiated with different polymer initiators: acid initiated polymer (■, OAX1), dodecanol initiated polymer (•, OAX3), and PEG-300 initiated polymer (▲, OAX4).

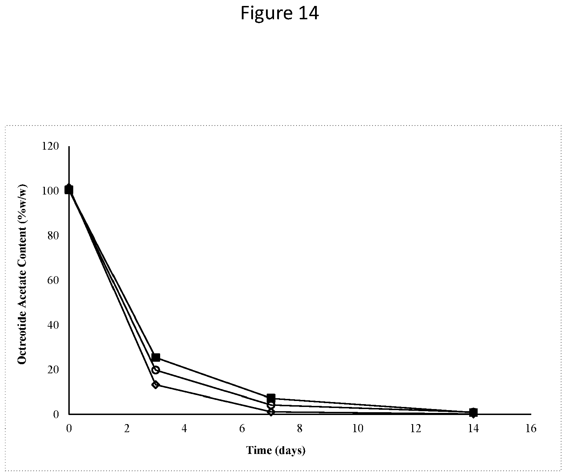

Fig. 14 shows the percent recovery of octreotide acetate (OA) as a function of time (days) at 37 °C for LPT formulations comprising an acid-initiated lactide:caprolactone copolymer (75:25) in the presence of magnesium acetate: 0.55 mg/mL ("low" concentration) of magnesium acetate (∘, OA1), 1.1 mg/mL ("high" concentration) of magnesium acetate (0, OA2), and control formulation containing no salt (■, OAX2).

### DETAILED DESCRIPTION OF THE INVENTION

The present invention generally relates to extended release pharmaceutical formulations that include biodegradable liquid polymers. Certain active pharmaceutical ingredients (APIs) such as peptide drugs are prone to chemical or physical degradation, including but not limited to acid degradation and acylation, in the presence of lactide- and/or glycolide-containing polymers (e.g., poly(lactide-co-glycolide) (PLG) polymers) used in pharmaceutical compositions of the prior art. The effect of poly(lactide-co-glycolide) (PLG) copolymers on the stability of a peptide molecule can be influenced by many factors such as monomer ratio, molecular weight, polydispersity index (PDI), polymer end group, solubility of the drug, properties of the solvent, and concentration of the polymer in solution. Accordingly, maintaining stability throughout the life-cycle of an extended release polymer-based product can be a significant challenge for drug molecules that are susceptible to acid degradation, such as peptide drugs. For example, pH will drop in the local environment, during hydrolytic degradation of a polymer implant, and this can result in peptide instability, which can be further exacerbated at higher temperatures, such as body temperatures. It can thus be especially difficult to formulate pharmaceutically acceptable compositions of these types of APIs that allow for extended release of the drug.

The present invention provides pharmaceutical compositions containing peptides that are suitable for, among other uses, extended release of the API upon administration to a patient. The present invention achieves this and other benefits by providing biodegradable, liquid polymer pharmaceutical compositions in which the stability of the peptide drug is improved by the selection of an appropriate liquid polymer system, and in some embodiments, by the further inclusion of a biocompatible solvent or combination or mixture of solvents and/or co-solvents and/or a divalent cation in the composition. When pharmaceutical compositions and formulations are provided using the liquid polymer system of the invention, according to the teachings provided herein, the peptide drug advantageously remains in a more stable chemical and physical form (*e.g.* shows less degradation due to acid degradation, acylation, etc.) during manufacture, transportation and storage of the formulation or *in vivo* within the body of the patient and when exposed to the internal environment of the body of the patient, for a longer period than has been achieved by the compositions of the prior art. The factors affecting the stability may include at least the composition of the peptide, the composition of the liquid polymer, and the presence (and, if present, identity and concentration) of a divalent cation within the composition.

Accordingly, the present invention includes biodegradable liquid polymer pharmaceutical compositions that can be administered into the body with syringes or needles and that are utilized to deliver a drug (active pharmaceutical ingredient, or API), and particularly a peptide or other polymeric drug, into the body over an extended period of time. Such compositions can deliver APIs to a patient at consistent levels within a therapeutic window for long periods of time to allow for improved ease of administration, resulting in improved patient compliance with administration protocols. In particular, the present invention is directed to liquid polymer pharmaceutical compositions and liquid polymer formulations that include a biodegradable polymer and an active pharmaceutical ingredient (API) that is characterized as a peptide (*i.e.*, a peptide drug), and that in some embodiments may further include a divalent cation and/or a solvent or combination or mixture of solvents and/or co-solvents. The liquid polymer formulations of the invention remain liquid after administration to the body (*e.g.* do not form solid implants *in vivo*, as discussed in detail herein) and remain stable with respect to both the polymer and the API over a wide range of temperatures.

It has been unexpectedly discovered that, in a liquid polymer delivery system that includes a biodegradable liquid polymer, and peptide drugs comprising at least one, two, three, four, five, six, seven, or more accessible amine groups, the peptides are susceptible to degradation due to exposure to the acidic groups and/or esters present on the liquid polymer, and that such peptides may be stabilized by utilizing a biodegradable liquid block copolymer that is initiated by a low-molecular weight polyethylene glycol (PEG) and/or comprises a polymer block comprising a low-molecular weight PEG. The stability of the peptide drugs may be further improved by the addition of divalent metal ions.

Further, it has been unexpectedly discovered that, in a liquid polymer delivery system that includes a biodegradable liquid polymer and peptide drugs comprising few, one or no accessible amine groups, the peptide drugs may be stabilized by the addition of divalent metal ions.

The liquid polymer systems of the present invention do not undergo reverse thermal gelation, *i.e*., they are not reverse thermal gels and do not have thermal gelation properties. Reverse thermal gels are known and described in the prior art, e.g. in U.S. Patent 6,201,072 to Rathi et al., U.S. Patent Application Publication 2007/0265356 to Kim et al., and U.S. Patent 9,901,554 to Bruin et al., the entireties of all of which are incorporated herein by reference. Reverse thermal gels, or polymers with thermal gelation properties, can be most simply described as polymers that, when heated from a lower temperature to a higher temperature, undergo a phase transition from liquid at the lower temperature to a gel at higher temperature. In other words, reverse thermal gels spontaneously increase in viscosity, and in many instances transform into a semisolid gel, as the temperature of the polymer solution is increased, *e*.*g*., above the gelation or phase transition temperature. Unlike reverse thermal gel polymers, the liquid polymers of the present invention are not susceptible to phase change or an increase in viscosity due to an increase in temperature, over a range of temperatures spanning at least room temperature and human body temperature, and the viscosity of such liquid polymer systems is substantially independent of temperature over this temperature range. Moreover, the liquid polymer compositions of the invention remain in liquid form *in vivo*, i.e. do not form a solid implant *in vivo*, even after the solvent has dissipated from the polymer upon exposure to the aqueous environment in the body.

In addition, polymers and copolymers with reverse thermal gelation properties are also often referred to as "hydrogels", because such polymers are typically miscible or soluble in water at temperatures below which the gelation phase transition occurs. In contrast, biodegradable liquid polymers of the present invention are not soluble in water.

As used herein, the term "accessible amine group" refers to an amine group that is open (e.g., sterically open) and available to react with or to perform nucleophilic attack on the ester backbone of a polymer or to perform an acid-base reaction with an available acid group in a polymer.

As used herein, the term "animal" refers to any organism of the kingdom Animalia. Examples of "animals" as that term is used herein include, but are not limited to, humans (*Homo sapiens*); companion animals, such as dogs, cats, and horses; and livestock animals, such as cattle, goats, sheep, and pigs.

As used herein, the term "biocompatible" means "not harmful to living tissue."

As used herein, the term "peptide" means a polymer formed by the covalent linkage of individual amino acids through the formation of a peptide bond. Peptides may be from 2 to 39 amino acids in length as well as being naturally occurring or synthetic in nature. Furthermore, peptides may be linear or cyclic in nature. Peptides may exist as independent compounds or be derived from larger proteins and may be unstructured in nature or display various levels of secondary protein structure, e.g. α-helix or β-sheet folding. Peptides may possess functional biological activity either alone or in combination with additional binding partners.

As used herein, the term "biodegradable" refers to any water-insoluble material that is converted under physiological conditions into one or more water-soluble materials, without regard to any specific degradation mechanism or process. Water-insoluble polymers that are converted under physiological conditions into one or more water-soluble materials are referred to as herein as "biodegradable polymers," and non-limiting examples of biodegradable polymers include polymers, co-polymers or terpolymers comprising: lactide, glycolide, caprolactone, trimethylene carbonate, or dioxanone monomers.

As used herein, the term "liquid" refers to the ability of a composition to undergo continuous deformation under a shearing stress, regardless of the presence or absence of a non-aqueous solvent. Liquid polymer compositions and the liquid polymers according to the invention have a liquid physical state at ambient and body temperatures and remain liquid *in vivo*, *i.e*., in a largely aqueous environment. The liquid polymer compositions and liquid polymers have a definite volume, but are an amorphous, non-crystalline mass with no definite shape. In addition, the liquid polymers according to the invention are not soluble in body fluid or water and therefore, after injection into the body and dissipation of the solvent, remain as a cohesive mass when injected into the body without themselves significantly dissipating. In addition, such liquid polymer compositions can have a viscosity, density, and flowability to allow delivery of the composition through standard gauge or small gauge needles (e.g., 18-26 gauge) with low to moderate injection force using standard syringes. The liquid polymers of the present invention can be further characterized as not forming a solid implant *in situ* in the body when injected into the body as part of a extended release drug delivery system that includes the liquid polymers and a biocompatible solvent or combination or mixture of solvents and/or co-solvents. In other words, liquid polymers according to the present invention remain in a substantially liquid form *in situ* upon exposure to an aqueous environment, such as upon injection into the body, including *after* the solvent in the administered composition has dissipated. The liquid polymers of the present invention can be further characterized as being non-crystalline, amorphous, non-thermoplastic, non-thermosetting, and/or non-solid. "Liquids," as that term is used herein, may also exhibit viscoelastic behavior, *i.e.* both viscous and elastic characteristics when undergoing deformation, such as time-dependent and/or hysteretic strain. By way of non-limiting example, viscoelastic materials that are generally flowable but have a partially gel-like character, such as cake batter or raw pizza dough, and similar materials, are "liquids" as that term is used herein. In some embodiments, materials having a non-zero yield stress that do not deform at stresses below the yield stress, and that are readily deformable without a characteristic of material fracture or rupture at stress above the yield stress, may be "liquids" as that term is used herein.

As used herein, the terms "low-molecular weight polyethylene glycol" and "low-molecular weight PEG," unless otherwise specified, refer to polyethylene glycols (PEGs) having a number average molecular weight of no more than 5,000 daltons. In block copolymers comprising both a PEG block and a block of one or more other monomers, the PEG block may be referred to as a "low-molecular weight polyethylene glycol" block or "low-molecular weight PEG" block if the number average molecular weight of the PEG block alone is no more than 5,000 daltons, regardless of the number or weight average molecular weight of the entire block copolymer.

As used herein, the terms "molecular weight" and "average molecular weight" can refer to number average molecular weight or weight average molecular weight. Generally, when referring to PEG blocks or PEG components herein, number average molecular weight is used (*e.g*., PEG300 is a PEG having a number average molecular weight of 300 daltons). Generally, when referring to a copolymer herein, including a di- or tri-block copolymer, weight average molecular weight is used. Reference to "number average molecular weight" or "Mₙ" refers to total number of molecules in a unit mass of polymer, and is calculated by dividing the total weight of polymer by the total number of molecules. "Weight-average molecular weight" or "Mw" depends not only on the number of molecules present, but also on the weight of each molecule. Therefore, Mw is determined by methods that are sensitive to the molecular size rather than just their number, such as light scattering techniques. As used herein weight average molecular weight of a polymer is the molecular weight as measured by a conventional gel permeation chromatography (GPC) instrument (such as an Agilent 1260 Infinity Quaternary LC with Agilent G1362A Refractive Index Detector) utilizing polystyrene standards and tetrahydrofuran (THF) as the solvent.

As used herein, the terms "patient" and "subject" are interchangeable and refer generally to an animal to which a composition or formulation of the invention is administered or is to be administered.

As used herein, the term "polymer" refers generally to polymers, copolymers and/or terpolymers that can be linear, branched, grafted and/or star-shaped. Non-limiting examples of polymers include polyglycolides, polylactides, polycaprolactones, polyanhydrides, polyorthoesters, polydioxanones, polyacetals, polyesteramides, polyamides, polyurethanes, polycarbonates, polyphosphazenes, polyketals, polyhydroxybutyrates, polyhydroxyvalerates, polyethylene glycols, polyesters, and polyalkylene oxalates, and copolymers or terpolymers comprising combinations of monomers thereof.

As used herein, the term "small molecule" means an organic compound having a molecular weight less than 900 daltons.

Unless otherwise specified, all ratios between monomers in a copolymer disclosed herein are molar ratios.

As used herein, the term "solvent" refers to a liquid that dissolves a solid or liquid solute, or to a liquid external phase of a suspension throughout which solid particles are dispersed.

### Liquid Polymers

The liquid polymer compositions of the invention comprise a biodegradable liquid polymer. The liquid polymers remain in a liquid (flowable) form , *i.e.* undergo continuous deformation under a shearing stress greater than zero and/or greater than a yield stress, at room temperature (*e.g*., at approximately 25°C) up to body temperature (*e.g*., at approximately 37°C) or higher, even after dissipation of the solvent from the polymer composition, such as when the polymer composition is exposed to an aqueous or largely aqueous environment (*e.g. in vivo*). The characteristic of being liquid is achieved by control of the molecular weight of the polymer and the monomer selection and ratio. In addition, the liquid polymer can have a pre-injection bulk viscosity that allows the composition to be easily administered, and in some embodiments effective to provide a desired extended release profile of a biologically active agent from the implanted material. Because the liquid polymers are liquid at room temperature, they allow the use of lower concentrations of the biocompatible solvent or combination or mixture of solvents and/or co-solvents to be used in the composition to provide a syringeable formulation compared to polymer/solvent compositions prepared with solid polymers.

Examples of suitable liquid polymers that can be used in this application include polylactic acid, polyglycolic acid, polylactide (D,L-lactide, D-lactide, L-lactide), polyglycolide, polycaprolactones, polyanhydrides, polyamides, polyurethanes, polyesteramides, polyorthoesters, polydioxanones, polyacetals, polyketals, polycarbonates, polyphosphazenes, polyhydroxybutyrates, polyhydroxyvalerates, polyalkylene oxalates, polyalkylene succinates, poly(malic acid), polyethylene glycol, hyaluronic acid, chitin and chitosan, and copolymers, terpolymers, and combinations or mixtures of the above materials. In one embodiment, the liquid polymer is selected from the group consisting of a polylactide, a polyglycolide, a polycaprolactone, a poly(trimethylene carbonate), a polydioxanone, a copolymer thereof, a terpolymer thereof, or any combination thereof. Preferred materials include those polymers, copolymers or terpolymers made with lactide, glycolide, caprolactone, p-dioxanone, trimethylene carbonate, 1,5-dioxepan-2-one, 1,4-dioxepan-2-one, ethylene oxide, propylene oxide, sebacic anhydride, and diketene acetals/diols, and lactic acid with lower molecular weights and amorphous regions to limit crystallinity and subsequent solidification.

Non-limiting examples of suitable liquid polymers according to the invention include copolymers of D,L-lactide, D-lactide, L-lactide or glycolide and ε-caprolactone with molar ratios of lactide (or glycolide)/caprolactone ranging from about 90:10 to about 10:90, and copolymers of D,L-lactide, D-lactide, L-lactide or glycolide and trimethylene carbonate (TMC) with molar ratios of lactide (or glycolide)/TMC ranging from about 90:10 to about 10:90. Generally, liquid polymers and liquid polymer compositions of the invention can have an inherent viscosity as determined in a 0.10 g/dL solution of hexafluoroisopropanol at 25° C from 0.05 to 0.50 dL/g.

In some embodiments, the liquid polymer may be a copolymer comprising a first monomer selected from the group consisting of lactide (D,L-lactide, D-lactide, and/or L-lactide), glycolide, and combinations thereof and a second monomer selected from the group consisting of caprolactone, TMC, and combinations thereof. In further embodiments, the liquid copolymer may be a block copolymer comprising a first block comprising a first monomer selected from lactide (D,L-lactide, D-lactide, and/or L-lactide), glycolide, and combinations thereof and a second monomer selected from caprolactone, TMC, and combinations thereof, and a second block comprising polyethylene glycol (PEG).

In embodiments of the composition, the biodegradable liquid polymer is a copolymer of two monomers having a molar ratio of any two whole numbers X to Y, where each of X and Y is at least about 10 and no more than about 90 and the sum of X and Y is 100. In embodiments of the composition, the biodegradable liquid polymer is a copolymer of two monomers having a molar ratio of any two whole numbers X to Y, where each of X and Y is at least about 25 and no more than about 75 and the sum of X and Y is 100. In some embodiments, the lactide or glycolide monomers and the caprolactone and/or TMC monomers are present in a molar ratio of between about 5:95 and about 95:5, between about 10:90 and about 90:10, between about 20:80 and about 80:20, between about 25:75 and about 75:25, or between about 30:70 and about 70:30. By way of non-limiting example, a first monomer of the two or more monomers may be selected from the group consisting of lactide, glycolide, and combinations thereof and a second monomer of the two or more monomers may be selected from the group consisting of caprolactone, TMC, and combinations thereof.

Further examples of suitable liquid polymers of the invention include biodegradable liquid polymers comprising a copolymer with lactide (including D,L-lactide, D-lactide, and/or L-lactide) and/or glycolide residues, wherein the molar percentage of the lactide and/or glycolide residues make up greater than about 5% and less than about 95%. In some embodiments, the lactide and/or glycolide monomer residues are present at at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% of total monomer residues of the copolymer. Other examples of suitable liquid polymers of the invention include biodegradable liquid polymers comprising a copolymer with caprolactone and/or trimethylene carbonate residues, wherein the caprolactone and/or trimethylene carbonate residues make up in an amount greater than about 5% and less than about 95%. In some embodiments, the caprolactone and/or trimethylene carbonate monomer residues are present at at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, or at least about 90% of total monomers of the copolymer. Further embodiments include liquid polymers having molar ratios of about 75:25 D,L-lactide:ε-caprolactone and of about 75:25 D,L-lactide:trimethylene carbonate.

In embodiments, biodegradable liquid polymers of the invention may comprise a polymer block comprising a low-molecular weight polyethylene glycol (PEG). Further, the polymers can have a ratio of ethylene glycol monomer units to monomer units other than ethylene glycol (e.g. lactide, glycolide, caprolactone, TMC, and combinations thereof) that is between about 5:95 and about 35:65, between about 10:90 and about 30:70, or between about 15:85 and about 25:75. The ratio of ethylene glycol monomer units to monomer units other than ethylene glycol may range from any whole number ratio to any other whole number ratio within the range of about 1:99 to about 40:60. In some embodiments, the ratio of ethylene glycol monomer units to monomer units other than ethylene glycol is about 10:90, about 20:80, or about 30:70.

In embodiments, the biodegradable liquid polymer may be formed using an initiator selected to provide a desired structure, and in particular a desired polymer block structure or end group structure, to the biodegradable liquid polymer. By way of non-limiting example, the polymer may be formed using a low-molecular weight PEG as an initiator, which may in embodiments result in the formation of a block copolymer comprising a low-molecular weight PEG block. By way of further non-limiting example, the polymer may be formed using an organic acid, e.g. a hydroxy acid such as glycolic acid, which may in embodiments result in the formation of a polymer comprising at least one carboxylic acid end group. As the following description and Examples set forth in greater detail, the initiator may be selected, whether alone or in combination with a selected divalent cation, to provide for improved stability of a peptide drug in the formulation; by way of non-limiting example, a low-molecular weight PEG-initiated polymer may be used with or without a divalent cation to stabilize a peptide having at least one accessible amine group, and an acid-initiated polymer may be used in combination with a divalent cation to stabilize a peptide having few or no accessible amine groups.

Biodegradable liquid polymers of the present invention are not reverse thermal gelling polymers. Reverse thermal gelling polymers are polymers that are soluble in water at a selected temperature (*e.g*. room temperature or below) and undergo a spontaneous phase transition, *i.e.* increase in viscosity, as temperature increases to form a physical gel at an elevated temperature (*e.g*. human body temperature). Unlike reverse thermal gels, biodegradable liquid polymers of the present invention have viscosities that, over at least a selected range of temperatures, are substantially independent of temperature and/or do not spontaneously increase with an increase in temperature, and in particular may remain liquid and/or flowable at both room temperature and human body temperature. In some embodiments, the non-reverse thermal gelling properties of biodegradable liquid polymers of the present invention may be achieved by providing a biodegradable liquid polymer having end groups that are not covalently modified, *e.g*. by aliphatic hydrocarbon groups or acyl groups.

Biodegradable liquid polymers of the present invention may comprise a block copolymer comprising at least two polymer blocks A and B. By way of non-limiting example, block A may comprise first monomer residues selected from D,L-lactide, D-lactide, L-lactide, glycolide, or combinations thereof and second monomer residues selected from ε-caprolactone, trimethylene carbonate, or combinations thereof. Block B comprises a low-molecular weight polyethylene glycol (PEG). The blocks may be arranged in any number or order (*e.g.* as a di-block copolymer A-B, or a tri-block copolymer A-B-A or B-A-B). Such polymers are formed by initiation of the first and second monomer residues with a low-molecular weight PEG initiator. The PEG block may, in some embodiments, comprise methoxy-PEG. In block copolymers comprising a low-molecular weight PEG block, a molar ratio of ethylene glycol monomers to all other monomers with the block copolymer may be at least about 5:95, or at least about 10:90, or at least about 20:80, or at least about 30:70, or at least about 40:60, or at least about 50:50, or any whole number ratio within the range of about 1:99 to about 60:40. In one embodiment, a molar ratio of ethylene glycol monomers to all other monomers with the block copolymer may be between about 10:90 and about 50:50. By way of non-limiting example, a molar ratio of first monomer (e.g. lactide and/or glycolide) to second monomer (e.g. caprolactone and/or TMC) to ethylene glycol in the polymer as a whole may be X:Y:Z, where X can be any number between about 25 and about 75, Y can be any number between about 5 and about 45, and Z can be any number between about 5 and about 55, such that the sum of X, Y, and Z is about 100. Table 1 illustrates non-limiting examples of polymers of this kind.

The PEG may have any number average molecular weight between about 200 daltons and about 5,000 daltons. In some embodiments, the number average molecular weight of the PEG may be about 4,500 daltons or less, or about 4000 daltons of less, or about 3500 daltons or less, or about 3000 daltons or less, or about 2500 daltons or less, or about 2000 daltons or less. In some embodiments, the number average molecular weight of the PEG may be about 1,900 daltons or less, or about 1,800 daltons or less, or about 1,700 daltons or less, or about 1,600 daltons or less, or about 1,500 daltons or less, or about 1,400 daltons or less, or about 1,300 daltons or less, or about 1,200 daltons or less, or about 1,100 daltons or less, or about 1,000 daltons or less, or about 900 daltons or less, or about 800 dalton or less s, or about 700 daltons or less, or about 600 daltons or less, or about 500 daltons or less, or about 400 daltons or less, or about 300 dalton or less, or about 200 daltons. In some embodiments, the molecular weight of the PEG may be about 900 daltons or less, or about 800 daltons or less, or about 700 daltons or less, or about 600 daltons or less, or about 500 daltons or less, or about 400 daltons or less, or about 300 daltons or less.

The biodegradable, liquid polymers (including both PEG-initiated and acid-initiated polymers) suitable for use in formulations according to the present invention may generally have a weight average molecular weight of between about 1 kDa and about 40 kDa, or between about 1 kDa and about 35 kDa, or between about 1 kDa and about 30 kDa, or between about 1 kDa and about 25 kDa, or between about 1 kDa and about 20 kDa, or any value, in whole number increments, between about 1 kDa and about 40 kDa. In embodiments, the polymer has a weight average molecular weight of at least about 2 kDa, or at least about 3 kDa, or at least about 4 kDa, or at least about 5 kDa, or at least about 6 kDa, or at least about 7 kDa, or at least about 8 kDa, or at least about 9 kDa, or at least about 10 kDa, or at least about 11 kDa, or at least about 12 kDa, or at least about 13 kDa, or at least about 14 kDa, or at least about 15 kDa, or at least about 16 kDa, or at least about 17 kDa, or at least about 18 kDa, or at least about 19 kDa, or at least about 20 kDa, or at least about 21 kDa, or at least about 22 kDa, or at least about 23 kDa, or at least about 24 kDa, or at least about 25 kDa, or at least about 26 kDa, or at least about 27 kDa, or at least about 28 kDa, or at least about 29 kDa, or at least about 30 kDa, or at least about 31 kDa, or at least about 32 kDa, or at least about 33 kDa, or at least about 34 kDa, or at least about 35 kDa.

In embodiments of the composition, the biodegradable liquid polymer may make up between about 0.1 wt% and about 50 wt% of the composition, or between about 5 wt% and about 45 wt% of the composition, or between about 10 wt% and about 40 wt% of the composition, or between about 15 wt% and about 35 wt% of the composition, or between about 20 wt% and about 30 wt% of the composition, or about 20 wt% of the composition, or about 25 wt% of the composition, or about 30 wt% of the composition. Alternatively, the biodegradable liquid polymer may make up any whole-number weight percentage of the formulation between about 1 wt% and about 50 wt%, or may make up a range from any whole-number weight percentage of the formulation to any other whole-number weight percentage of the formulation with end points between 1 wt% and 50 wt%.

### Solvents

Solvents and co-solvents that may be used according to the invention are biocompatible, non-toxic, solvents, which may be either hydrophilic or hydrophobic solvents, or may be a combination of hydrophilic solvents, hydrophobic solvents or hydrophilic and hydrophobic solvents, depending upon the desired release profile and the solubility of the polymer and/or biologically active agent in the polymer/solvent composition. Solvents and co-solvents suitable for use in embodiments of the present invention include, by way of non-limiting example, acetone, benzyl benzoate, butyrolactone, ε-caprolactone, N-cycylohexyl-2-pyrrolidone, diethylene glycol monomethyl ether, dimethyl acetamide, dimethyl formamide, dimethyl sulfoxide (DMSO), ethyl acetate, ethyl lactate, N-ethyl-2-pyrrolidone, glycerol formal, glycofurol, N-hydroxyethyl-2-pyrrolidone, isopropylidene glycerol, lactic acid, methoxypolyethylene glycol, methoxypropylene glycol, methyl acetate, methyl ethyl ketone, methyl lactate, N-methyl-2-pyrrolidone (NMP), low-molecular weight (MW) polyethylene glycol (PEG), polysorbate 80, polysorbate 60, polysorbate 40, polysorbate 20, polyoxyl 35 hydrogenated castor oil, polyoxyl 40 hydrogenated castor oil, sorbitan monolaurate, sorbitan monostearate, sorbitan monooleate, benzyl alcohol, isopropanol, tert-butanol, n-propanol, propylene glycol, 2-pyrrolidone, α-tocopherol, triacetin, tributyl citrate, acetyl tributyl citrate, acetyl triethyl citrate, triethyl citrate, furfural, esters thereof, and combinations thereof.

The biocompatible solvent, or combination or mixture of solvents and/or co-solvents used in a composition of the invention, will generally comprise between about 20 wt% and about 95 wt% of the formulation, or between about 35 wt% and about 80 wt% of the formulation, or between about 45 wt% and about 65 wt% of the formulation, or between about 45 wt% and about 55 wt% of the formulation, or about 50 wt% of the formulation, or alternatively the solvent or combination or mixture of solvents and/or co-solvents can range from any whole number percentage by weight of the formulation to any other whole number percentage by weight of the formulation between about 20 wt% and about 95 wt%.

In one embodiment, the liquid polymer composition comprises between about 20 wt% and about 50 wt% biodegradable liquid polymer and between about 50 wt% and about 70 wt% biocompatible solvent. The liquid polymer/solvent concentrations permit the liquid polymer/solvent compositions to be easily injected with standard syringes and small gauge needles (e.g., about 18-26 gauge). The compositions of the invention can be administered into the body of a human subject or other animal such as a dog, cat, horse, cow, goat, sheep, or pig.

### Divalent Metal Ions

Pharmaceutical compositions according to the present invention may further comprise a divalent metal ion or cation selected to improve the stability of a peptide drug in the pharmaceutical formulation. Commonly, the divalent cation will be selected from the group consisting of the alkaline earth metals and the transition metals normally having a +2 oxidation state, and most commonly will be selected from the group consisting of magnesium, calcium, and zinc. The divalent cation may, in embodiments, be provided as a metal salt, i.e. in conjunction with an anion; by way of non-limiting example, the anion may be selected from the group consisting of acetate and chloride, and thus the metal salt may be the acetate or chloride of the selected metal (e.g. the acetate or chloride of magnesium, calcium, or zinc). Because each divalent cation and/or metal salt may have a differing effect on the stability of a particular peptide, the divalent cation and/or metal salt may be selected to provide enhanced stability based on the peptide desired to be incorporated into the formulation, as is further described in the description and Examples below.

The concentration of a metal salt comprising a divalent cation in the pharmaceutical composition may be selected based on various factors described herein, including but not limited to the stability-enhancing effect of the metal salt on the peptide of interest and a pharmaceutically acceptable or pharmaceutically recommended limit on the concentration of the metal salt in the pharmaceutical composition.

As a first non-limiting example, where the metal salt is magnesium acetate, the magnesium acetate may have a concentration in the pharmaceutical composition of between about 0.01 mg/mL and about 2.75 mg/mL, or between about 0.25 mg/mL and about 2.5 mg/mL, or between about 0.5 mg/mL and about 2.25 mg/mL.

As a second non-limiting example, where the metal salt is magnesium chloride, the magnesium chloride may have a concentration in the pharmaceutical formulation of between about 0.01 mg/mL and about 3.75 mg/mL, or between about 0.6 mg/mL and about 3.15 mg/mL, or between about 1.2 mg/mL and about 2.55 mg/mL.

As a third non-limiting example, where the metal salt is calcium chloride, the calcium chloride may have a concentration in the pharmaceutical formulation of between about 0.01 mg/mL and about 1.6 mg/mL, or between about 0.4 mg/mL and about 1.2 mg/mL, or between about 0.75 mg/mL and about 0.85 mg/mL.

As a fourth non-limiting example, where the metal salt is zinc acetate, the zinc acetate may have a concentration in the pharmaceutical formulation of between about 0.01 mg/mL and about 8.2 mg/mL, or between about 1.05 mg/mL and about 6.75 mg/mL, or between about 2.1 mg/mL and about 5.7 mg/mL.

As a fifth non-limiting example, where the metal salt is zinc chloride, the zinc chloride may have a concentration in the pharmaceutical formulation of between about 0.01 mg/mL and about 1.4 mg/mL, or between about 0.05 mg/mL and about 1.2 mg/mL, or between about 0.1 mg/mL and about 1.05 mg/mL.

### Administration

The liquid polymer and solvent composition with an active pharmaceutical agent can be applied or injected into the body of a subject or onto an object (e.g., mesh, catheter, a screw, plate, tack, pin, staple, sponge, etc.) using a device such as a syringe or needle. A device with the composition thereon can be placed into the body of the subject. Suitable routes of injection include, but are not limited to, any parenteral route, such as subcutaneous, intramuscular, intraarticular, and intradermal routes. Other routes of administration include, but are not limited to, topical administration, or sublingual administration (such as on a film or similar system). By way of non-limiting example, liquid polymer formulations of the invention may be administered in an articular region, a cutaneous region, an ocular region, and/or a tumor site region where it is desired that the API act locally rather than systemically. One advantage of formulations of the invention is that, due to the liquid nature of the formulation, they form an implant or depot that can be characterized as soft, malleable, non-rigid, and/or non-solid. Such formulations, when administered into, for example, an articular region or other region where mobility or sensitivity may be an issue, result in the formation of an implant or depot *in vivo* after administration that has physical characteristics that allow the implant or depot to be better tolerated and have much less impact on mobility than, for example, a solid, hard implant. Following injection or use, the solvent dissipates to leave a liquid bolus of polymer and active pharmaceutical agent. The liquid polymer component of the implanted polymer/solvent compositions of the invention will flow and fill the voids left by the solvent as it dissipates from the implanted material. The implanted liquid polymer material remains as a liquid with a fluctuant (flowable) consistency so that it remains movable and compressible and gradually biodegrades in the subject's body over time. Because the liquid polymer is not soluble in body fluid or water, the bolus within the body tissue does not dissipate by dissolution in body fluid, but remains as a cohesive mass.

The liquid polymer/solvent compositions can be used as extended release compositions to provide a delivery system in which a drug or other biologically active agent is added to the liquid polymer/solvent composition to form a liquid polymer pharmaceutical composition prior to injection or other route of administration to the body. Upon exposure to body fluid, the solvent dissolves or dissipates in the aqueous tissue fluid to leave the more viscous liquid polymer for release of the encapsulated or entrapped active agent. The liquid polymer implant formed from compositions of the present invention by the dissolution or dissipation of the solvent can be used to control the release of biologically active agents with low initial burst and extended release of the drug.

Pharmaceutical compositions according to the present invention may be provided as a component of a delivery system comprising a syringe, wherein the pharmaceutical composition is contained within the syringe. Accordingly, such delivery systems are likewise within the scope of the present invention. In some embodiments, the syringe may be an autoinjector.

Liquid polymer pharmaceutical compositions of the invention have been found to release drugs in a patient over a surprisingly long period of time, release a surprisingly high total amount of drug and allow for modulation of initial burst profiles to achieve a high or low initial burst, as desired. In various embodiments, an active pharmaceutical agent in a liquid polymer pharmaceutical composition of the invention is released in a patient, for example as determined by measuring blood serum levels of the agent in a patient, for greater than three days, greater than one week, greater than two weeks, greater than three weeks, greater than four weeks, greater than one month, greater than two months, greater than three months, greater than four months, greater than five months, greater than six months, greater than nine months, or greater than one year. Such levels of agent can be at levels having a pharmacologic or therapeutic effect.

An embodiment of the present invention is a method to treat, provide a therapy for, cure, or prevent a disease, disorder, or other ailment by administration of a liquid polymer pharmaceutical composition comprising an active pharmaceutical agent, as described in detail elsewhere herein. These methods can be used to treat, provide a therapy for, cure, or prevent microbial infections or any other infections by a pathogen, autoimmune disorders, allergies, inflammations, cancers, endocrine disorders, metabolic disorders, neurological disorders, motor disorders, psychological disorders, cardiovascular disorders, or any other diseases, disorders, or other ailments treated by the active pharmaceutical agents described herein.

### Peptides

Active pharmaceutical agents (e.g., peptide drugs) that are suitable for the present application are biologically active agents that provide a biological effect and that act locally or systemically in the treatment, therapy, cure and/or prevention of a disease, disorder, or other ailment. Examples of drugs include, without limitation, peptide drugs, such as a peptide, polypeptide, or protein. Examples of peptide and polymeric drugs that are suitable for the present application include degarelix, abaloparatide, teriparatide, leuprolide (leuprorelin), dulaglutide, basal insulin, goserelin, triptorelin, nafarelin, buserelin, histrelin, deslorelin, ganirelix, abarelix, teverelix, lanreotide, carfilzomib, human growth hormone, interferon-alpha, interferon-beta, interferon-gamma, interleukin, growth hormone releasing peptides, glucagon-like peptides, granulocyte-colony stimulating factor, nerve growth factor, platelet-derived growth factor, insulin-like growth factor, vascular endothelial growth factor, fibroblast growth factor, octreotide, bone morphogenic protein, erythropoietin, albiglutide, anaritide, angiotensin II, buforin II, calcitonin, carperitide, cecropin P1, cetrorelix, dermaseptin, desmopressin, drosocin, enfuvirtide, etelcalcetide, exenatide, indolicidin, liraglutide, lixisenatide, magainin I, magainin II, nesiritide, neurotensin, pramlintide, ranalexin, semaglutide, tachyplesin, teduglutide, vasopressin, 1-deamino-8-D-arginine vasopressin, and salts, complexes, prodrugs, and analogs thereof.

A suitable drug that may be utilized in the present invention is abaloparatide. Abaloparatide is a parathyroid hormone-related protein (PTHrP) analog drug that has attracted interest as a potential treatment for osteoporosis. Like the related drug teriparatide, but unlike bisphosphonates, abaloparatide is an anabolic (i.e., bone-growing) agent. Further embodiments of the invention include liquid polymer pharmaceutical compositions of abaloparatide and use thereof in the treatment of osteoporosis by administration to a patient having osteoporosis in amounts and dosing schedules and by routes of administration as disclosed elsewhere herein, which may include, without limitation, intermittent doses as needed and prescribed by a physician.

Another suitable drug that may be utilized in the present invention is leuprolide or an acceptable salt thereof. Leuprolide, also known as leuprorelin, is a gonadotropin-releasing hormone analog (also known as a luteinizing hormone-releasing hormone (LHRH) agonist) that has found uses, by way of non-limiting example, in the treatment of various forms of cancer, particularly prostate and breast cancers, as well as endometriosis, uterine fibroids, and central precocious puberty (CPP). By way of example, treatment of advanced prostate cancer with leuprolide acetate may consist of 7.5 mg subcutaneous doses administered monthly, 22.5 mg subcutaneous doses administered every three months, 30 mg subcutaneous doses administered every four months, or 45 mg subcutaneous doses administered every six months. By way of example, treatment of endometriosis with leuprolide acetate may consist of 3.75 mg subcutaneous doses administered monthly for up to six months, or two 11.25 mg doses administered at three-month intervals. By way of example, treatment of uterine fibroids with leuprolide acetate may consist of 3.75 mg intramuscular doses administered monthly for up to three months, or a single 11.25 mg intramuscular dose. Further embodiments of the invention include liquid polymer pharmaceutical compositions of leuprolide or esters thereof and use thereof in the treatment of cancer (including prostate and breast cancer), endometriosis, uterine fibroids, or CPP by administration to a patient in need thereof in amounts and dosing schedules described above and by routes of administration as disclosed elsewhere herein.

Embodiments of the present invention include pharmaceutical compositions in which a peptide drug comprising at least one accessible amine groups (e.g. arginine, histidine, lysine, N-terminus, etc.) is stabilized by utilizing a biodegradable liquid polymer that is initiated by a low-molecular weight PEG and/or comprises a low-molecular weight PEG block, alone or in combination with a divalent cation. Without wishing to be bound by theory, it is believed that PEG-initiated polymers may retard acylation of the accessible amine group relative to, e.g., acid-initiated or dodecanol-initiated polymers. Peptides used in these embodiments may include any peptide comprising an N-terminal amine group and/or any natural or non-natural amino acid including an accessible amine group; such amino acids include but are not limited to arginine, histidine, lysine, L-2-amino-3-guanidinopropionic acid, 4-guanidinobutyric acid, Fmoc-Lys(Me,Boc)-OH, Fmoc-Lys(Me)₃-OH chloride, Fmoc-Lys(palmitoyl)-OH, and DL-5-hydroxylysine hydrochloride. In some embodiments, the peptide drug may comprise at least two accessible amine groups. In some embodiments, the peptide drug may comprise at least two, three, four, five, six, seven, eight, nine, ten, or more amino acids selected from arginine, histidine, lysine, L-2-amino-3-guanidinopropionic acid, 4-guanidinobutyric acid, Fmoc-Lys(Me,Boc)-OH, Fmoc-Lys(Me)₃-OH chloride, Fmoc-Lys(palmitoyl)-OH, and DL-5-hydroxylysine hydrochloride, and combinations thereof, and may additionally have an N-terminal amine group.

Accordingly, peptides with accessible amine groups that are suitable for use in these embodiments may include but are not limited to abaloparatide, albiglutide, anaritide, angiotensin II, buforin II, calcitonin, carperitide, cecropin P1, cetrorelix, dermaseptin, desmopressin, drosocin, enfuvirtide, etelcalcetide, exenatide, indolicidin, liraglutide, lixisenatide, magainin I, magainin II, nesiritide, neurotensin, pramlintide, ranalexin, semaglutide, tachyplesin, teduglutide, teriparatide, vasopressin, 1-deamino-8-D-arginine vasopressin, and pharmaceutically acceptable esters, salts, complexes, prodrugs, and analogs thereof.

Embodiments of the present invention include pharmaceutical compositions in which a peptide drug comprising few or no accessible amine groups is stabilized by utilizing a biodegradable liquid polymer that is initiated by an organic acid (e.g. a hydroxy acid such as glycolic acid) and/or comprises a carboxylic acid end group, in combination with a divalent cation. Peptides suitable for use in these embodiments may include but are not limited to linear peptides (e.g. leuprolide), cyclic peptides (e.g. lanreotide, octreotide), and pharmaceutically acceptable esters, salts, complexes, prodrugs, and analogs thereof, and which have a few, or one, or no accessible amine groups.

The concentration of active pharmaceutical agent in compositions of the invention depends on the drug that is included in the composition and may range from 0.1% to 50% by weight of the composition, including any whole number percent to any other whole number percent within the range of from about 1 percent to about 50 percent by weight. In some embodiments, the concentration of the active pharmaceutical agent is no more than about 25% by weight. Typically, for peptide drugs, the concentration of agent in the composition is between about 0.1% and about 15% by weight of the composition, or between about 0.1% and about 14% by weight of the composition, or between about 0.1% and about 13% by weight of the composition, or between about 0.1% and about 12% by weight of the composition, or between about 0.1% and about 11% by weight of the composition, or between about 0.1% and about 10% by weight of the composition, or between about 0.1% and about 9% by weight of the composition, or between about 0.1% and about 8% by weight of the composition, or between about 0.1% and about 7% by weight of the composition, or between about 0.1% and about 6% by weight of the composition, or between about 0.1% and about 5% by weight of the composition, or between about 0.1% and about 4% by weight of the composition, or between about 0.1% and about 3% by weight of the composition, or between about 0.1% and about 2% by weight of the composition, or between about 0.1% and about 1% by weight of the composition, or between about 0.1% and about 0.9% by weight of the composition, or between about 0.1% and about 0.8% by weight of the composition,. or between about 0.1% and about 0.7% by weight of the composition, or between about 0.1% and about 0.6% by weight of the composition, or between about 0.1% and about 0.5% by weight of the composition, or between about 0.1% and about 0.4% by weight of the composition, or between about 0.1% and about 0.3% by weight of the composition, or between about 0.1% and about 0.2% by weight of the composition, including any increment of 0.01% by weight between about 0.1% and about 15% by weight of the composition. In other embodiments, the amount of active pharmaceutical agent in compositions of the invention can range from any tenth of a percent to any other tenth of a percent within the range of from about 0.1 percent to about 50 percent by weight.

Because a beneficial characteristic of the compositions disclosed herein is improved extended release of an active pharmaceutical agent, the amount of active pharmaceutical agent will be suitable for long term treatment with the agent in accordance with the time frames disclosed herein. Other embodiments of the invention include single dosage formulations of the liquid polymer pharmaceutical composition which include the liquid polymer composition as described herein with an amount of an active pharmaceutical agent suitable for extended release. For example, such single dosage formulations can include sufficient active pharmaceutical agent for treatment of a patient for greater than three days, greater than one week, greater than two weeks, greater than three weeks, greater than four weeks, greater than one month, greater than two months, greater than three months, greater than four months, greater than five months, greater than six months, greater than nine months, or greater than one year. Compositions may be administered repeatedly as needed (e.g. every month, every three months, every six months, etc.).

The active pharmaceutical agent may be in the form of a liquid or a finely divided solid that is either dissolved or dispersed in the liquid polymer/solvent composition. The agent is incorporated into the composition in an amount sufficient to achieve the desired therapeutic effect, the desired release profile, and the desired period of release. There is no critical upper limit on the amount of the agent that is dispersed or dissolved in the liquid polymer/solvent solution as long as the solution has a fluid viscosity at room temperature acceptable for injection through a standard or small gauge syringe needle (e.g., gauge of 18-26). The lower limit of the amount of the agent incorporated into the liquid polymer/solvent solution is dependent upon the activity of the agent, the release rate needed to achieve the desired therapeutic level, and the length of time for treatment. Both soluble and insoluble active pharmaceutical agents may be incorporated into the liquid polymer/solvent system.

Liquid polymer/solvent compositions comprising an active pharmaceutical agent according to the present invention may appropriately be either a "solution" or a "dispersion" or a "suspension" of the active pharmaceutical agent in the biodegradable polymer and solvent. Particularly, it is to be understood that although liquid polymer is dissolved in the solvent, the active pharmaceutical agent may either be dissolved in the polymer and solvent (as in a solution), or form solid particles sufficiently large for suspension and sedimentation as part of a heterogeneous mixture (as in a suspension).

The compositions may include various adjuvants or additives, such as colorants, diluents, carriers, excipients, buffers, antioxidants, crystallization inhibitors, pH modifiers, and stabilizers.

The inventors have discovered that the stability of a peptide drug in a composition containing a liquid polymer delivery system according to the present invention that includes a biodegradable liquid polymer is markedly improved. As described herein, the stability may be markedly improved beyond that which is obtained when utilizing a similar liquid polymer delivery system that differs in the identity of the initiator end group of the biodegradable liquid polymer, and the stability may be markedly improved by including in the liquid polymer delivery system a divalent cation, e.g. a metal salt.

The inventors have discovered that the stability of a peptide drug comprising an accessible amine group in a composition containing a liquid polymer delivery system according to the present invention that includes a biodegradable liquid polymer is markedly improved by the use of a low-molecular weight polyethylene glycol (PEG) initiator to initiate the biodegradable liquid polymer. The low-molecular weight PEG is incorporated into the resulting biodegradable liquid polymer as a polymer block to form a block copolymer. The inventors have further discovered that the stability of the peptide drug may, but need not, be further improved by including in the composition a divalent metal cation selected from the group consisting of magnesium, calcium or zinc, and/or a metal salt selected from the group consisting of magnesium chloride, calcium chloride, and zinc acetate.

The inventors have discovered that the stability of a linear peptide drug comprising few or no accessible amine groups in a composition containing a liquid polymer delivery system according to the present invention that includes a biodegradable liquid polymer is markedly improved by including in the composition a divalent metal cation and/or a metal salt, and particularly a divalent metal cation selected from the group consisting of magnesium and zinc and/or a metal salt selected from the group consisting of magnesium acetate, zinc chloride, and zinc acetate.

The inventors have discovered that the stability of a cyclic peptide drug comprising few or no accessible amine groups in a composition containing a liquid polymer delivery system according to the present invention that includes a biodegradable liquid polymer is markedly improved by including in the composition a divalent metal cation and/or a metal salt, and particularly a divalent zinc cation and/or a metal salt selected from the group consisting of zinc chloride and zinc acetate.

The invention is illustrated by the following non-limiting examples.

### EXAMPLES

### Example 1: Methods of Forming LPT Formulations Comprising Peptides

The following example describes the preparation and test methods for Liquid Polymer Technology (LPT) formulations comprising various peptide molecules, and also describes the production and properties of a large panel of PEG-initiated liquid polymers according to the present invention.

### LPT Polymers

To produce the LPT formulations described in Examples 1-10 below, exemplary LPT polymers were produced using one of three different initiators. Specifically, LPT polymers were initiated using 1) glycolic acid as an initiator (*i.e.*, "acid-initiated polymers"), 2) dodecanol as an initiator (*i.e.*, "dodecanol-initiated polymers"), or 3) low-molecular weight polyethylene glycol (PEG) (*i.e*., "PEG-initiated polymers").

For example, to produce the poly(D,L-lactide-ε-caprolactone) (PDLCL) liquid copolymers, D,L-lactide, ε-caprolactone, and the selected initiator were provided in an amount calculated to achieve the target molar ratio in the composition and the target weight average molecular weight. Briefly, a 500 mL 2-part glass reactor equipped with a nitrogen inlet, an overhead stirrer with a vacuum-capable stir guide and a vacuum outlet leading to a vacuum trap and vacuum pump was assembled and placed in an oil bath. The oil bath was set at 100 °C and the reactor was placed under vacuum to remove any residual moisture.

For polymer compositions initiated with an acid initiator or dodecanol, the vacuum on the reactor was broken with nitrogen and the reactor was charged with the prescribed amounts of D,L-lactide, initiator, and ε-caprolactone via a glass funnel. For polymer compositions initiated with PEG, the reactor is charged with the PEG prior to adding the D,L-lactide and ε-caprolactone, and the vacuum is purged to remove moisture in the PEG. Following this step, the vacuum on the reactor was broken with nitrogen and the reactor was charged with the prescribed amounts of D,L-lactide and ε-caprolactone. For all three polymer compositions, at this stage, the stirrer was turned to 10-50 rpm, the oil bath set to 160°C, and the system vacuum purged and back flushed with nitrogen three times. The reactor was then left under a slight nitrogen purge.

A catalyst solution was prepared by weighing the appropriate amount of tin(II) 2-ethylhexanoate (stannous octoate) into a 10 mL volumetric flask and diluting to the mark with anhydrous toluene. For all polymer compositions described in these Examples, once the monomers had melted and the oil bath reached 160°C, typically 5 mL of the catalyst solution was injected in an amount calculated to achieve 0.03 wt% catalyst solution based on the monomer weight via a syringe equipped with a 6-inch blunt tipped 20 G needle with continuous stirring. By way of example, for a 400 gm batch of polymer, the amount of catalyst solution needed to add 0.03 wt% stannous octoate based on monomer weight was calculated as 0.12 g (in 5 mL of toluene). Also by way of example, for a 500 gm batch of polymer, the amount needed to add 0.03 wt% stannous octoate based on monomer weight was calculated as 0.15 g (in 5 mL of toluene).

After injection of the catalyst solution, the polymerization reaction was continued for 16-18 hours. After the appropriate reaction time, the vacuum trap was immersed in an ice bath and the nitrogen inlet closed. Vacuum was applied slowly to the stirred reaction mix for 4-6 hours with an ultimate vacuum of -22 to -25 in. Hg. Unreacted monomer was collected in the vacuum trap. After the appropriate time the vacuum was discontinued, the reactor purged with nitrogen, removed from the oil bath and the liquid polymer poured into a metal, glass or PYREX^{®} (low-thermal-expansion plastic borosilicate glass) container and cooled. The yield was approximately 85% for all polymer compositions.

The weight average molecular weight of the polymers was determined by gel permeation chromatography (GPC) with a refractive index detector (*e.g*., Agilent 1260 Infinity Quaternary LC with Agilent G1362A Refractive Index Detector).

Table 1 describes 17 different liquid, PEG-initiated block copolymers of the present invention that were produced to have various monomer molar ratios and target weight average molecular weights. Each of the polymers described in Table 1 is a block copolymer having an A-B-A structure, where "A" is a poly(D,L-lactide-ε-caprolactone) copolymer and "B" is a low-molecular weight PEG (exemplifying a variety of different low-molecular weight PEG initiators). The molar ratio of D,L-lactide to ε-caprolactone in each polymer was approximately 75:25. The exact quantities of monomer and initiator may vary when different lots of monomer are used, or when different initiators are used, and the appropriate amounts to achieve a given target molar ratio and molecular weight can be calculated by one of skill in the art using the guidance provided herein. For each of the biodegradable, liquid, PEG-initiated block copolymers described in Table 1, the table provides: (1) moles of D,L-lactide in the copolymer; (2) moles of ε-caprolactone (CL) in the copolymer; (3) identity of the specific low-molecular weight PEG initiator (Initiator); (4) moles of PEG in the copolymer (as well as the moles of ethylene glycol (EG) in the copolymer); (5) the molar percentage of D,L-lactide:ε-caprolactone:EG monomers (DL:CL:EG) in each copolymer; (6) the molar ratio of D,L-lactide and ε-caprolactone monomers to EG monomers ((DL+CL/EG) in each copolymer; (7) and the weight average molecular weight of the final block copolymer (kDa). In addition, for each of the block copolymers in Table 1, as shown in the table, notably, all were "liquid" polymers as defined by the present invention (Physical Form) meaning that the polymers remain in a liquid form as defined herein, even subsequent to injection and dissipation of the solvent, none were soluble in water (Solubility in Water), and none had thermogelling properties (Thermogel Property). Selected PEG-initiated copolymers from Table 1 were used in the Examples below.

The acid-initiated polymers used in the Examples below were glycolic acid-initiated, 75:25 poly(DL-lactide-co-ε-caprolactone) (PDLCL) liquid polymers (*i.e*., polymers comprised of 75% DL-lactide and 25% ε-caprolactone (mol:mol)), having various molecular weights as indicated in the individual Examples. By way of example, a 75:25 acid-initiated PDLCL copolymer having a weight average molecular weight of approximately 5 kDa can be produced using 2.2 moles D,L-lactide and 0.73 moles ε-caprolactone, initiated with 0.42 moles glycolic acid. The exact quantities of monomer and initiator may vary when different lots of monomer are used, or when different initiators are used, and the appropriate amounts to achieve a given target molar ratio and molecular weight can be calculated by one of skill in the art.

The dodecanol-initiated polymers used in the Examples below were all dodecanol-initiated, 75:25 poly(DL-lactide-co-ε-caprolactone) (PDLCL) liquid polymers (*i.e*., polymers comprised of 75% DL-lactide and 25% ε-caprolactone (mol:mol)), having various molecular weights as indicated. By way of example, a 75:25 dodecanol-initiated PDLCL copolymer having a weight average molecular weight of approximately 4 kDa can be produced using 1.65 moles D,L-lactide and 0.55 moles ε-caprolactone, initiated with 0.18 moles dodecanol. As discussed above, the exact quantities of monomer and initiator may vary when different lots of monomer are used, or when different initiators are used, and the appropriate amounts to achieve a given target molar ratio and molecular weight can be calculated by one of skill in the art.

**Table 1**

| No. | Polymer Description | DL-Lactide (mole) | CL (mole) | Initiator | PEG (mole) | EG (mole) | DL:CL:EG (Mole %) | (DL+CL)/E G (Molar Ratio) | Weight Average Mw (kDa) | Physical Form | Solubility in Water | Thermogel Property |
|---|---|---|---|---|---|---|---|---|---|---|---|---|
| 1 | 75/25 PDLCL-PEG200 | 1.37 | 0.46 | PEG200 | 0.05 | 0.227 | 67:23:10 | 9.0 | 10.7 | Liquid | No | No |
| 2 | 75/25 PDLCL-PEG400 | 1.38 | 0.47 | PEG400 | 0.05 | 0.454 | 61:19:20 | 4.0 | 9.8 | Liquid | No | No |
| 3 | 75/25 PDLCL-PEG200 | 1.65 | 0.55 | PEG200 | 0.113 | 0.513 | 62:21:17 | 4.9 | 5.8 | Liquid | No | No |
| 4 | 75/25 PDLCL-PEG1500 | 1.92 | 0.64 | PEG1500 | 0.07 | 2.384 | 39:13:48 | 1.1 | 9.3 | Liquid | No | No |
| 5 | 75/25 PDLCL-PEG200 | 1.37 | 0.46 | PEG200 | 0.051 | 0.232 | NA | NA | 10.0 | Liquid | No | No |
| 6 | 75/25 PDLCL-PEG400 | 1.37 | 0.44 | PEG400 | 0.048 | 0.436 | 60:19:21 | 3.8 | 11.7 | Liquid | No | No |
| 7 | 75/25 PDLCL-PEG300 | 1.37 | 0.46 | PEG300 | 0.067 | 0.456 | 58:21:21 | 3.8 | 7.7 | Liquid | No | No |
| 8 | 75/25 PDLCL-PEG400 | 3.29 | 1.1 | PEG400 | 0.098 | 0.890 | 59.8:22.1:18.2 | 4.5 | 11.7 | Liquid | No | No |
| 9 | 75/25 PDLCL-PEG300 | 1.65 | 0.55 | PEG300 | 0.08 | 0.545 | 57:21.3:21.7 | 3.6 | 7.9 | Liquid | No | No |
| 10 | 75/25 PDLCL-PEG300 | 3.02 | 1.01 | PEG300 | 0.147 | 1.001 | 57.3:21.3:21.4 | 3.7 | 8.3 | Liquid | No | No |
| 11 | 75/25 PDLCL-PEG300 | 1.65 | 0.55 | PEG300 | 0.056 | 0.381 | 55.3:20.5:24.1 | 3.1 | 11.7 | Liquid | No | No |
| 12 | 75/25 PDLCL-PEG300 | 1.65 | 0.55 | PEG300 | 0.104 | 0.708 | 49.3:18.2:32.6 | 2.1 | 6.6 | Liquid | No | No |
| 13 | 75/25 PDLCL-PEG300 | 1.65 | 0.55 | PEG300 | 0.048 | 0.327 | 57:21.1:21.9 | 3.6 | 13.9 | Liquid | No | No |
| 14 | 75/25 PDLCL-PEG300 | 1.65 | 0.55 | PEG300 | 0.035 | 0.238 | 65.4:24:10.7 | 8.4 | 17.0 | Liquid | No | No |
| 15 | 75:25 PDLCL-PEG300 | 1.65 | 0.55 | PEG300 | 0.27 | 1.839 | 31.3:37.2:31.5 | 2.2 | 4.8 | Liquid | No | No |
| 16 | 75:25 PDLCL-PEG300 | 1.65 | 0.55 | PEG300 | 0.27 | 1.839 | 36.8:16:47.2 | 1.1 | 2.5 | Liquid | No | No |
| 17 | 75:25 PDLCL-PEG600 | 1.65 | 0.55 | PEG600 | 0.1375 | 1.873 | 37.1:14.5:48.4 | 1.1 | 4.5 | Liquid | No | No |

*LPT Polymers Solutions.* LPT polymer solutions containing about 40% w/w of the various LPT polymers were combined with approximately 60% w/w of the solvent, N-methyl-2-pyrrolidone (NMP). For example, Table 2 provides relative amounts of the polymer and solvent, N-methyl-2-pyrrolidone (NMP), for three PDLCL polymers with molar ratio of D,L-lactide and ε-caprolactone of 75:25 which were used in some of the Examples herein. In the case of the PEG300-initiated polymer, the molar ratio of D,L-lactide to ε-caprolactone to ethylene glycol was about 49.3: 18.2:32.6 (see Table 1, Sample No. 12). The solutions were prepared by weighing materials into a 100 g FlackTek jar and mixing using a roller mixer. The solutions were mixed overnight and checked for any lumps using a spatula. Mixing was continued until the solutions were homogeneous and lump-free. Solutions were stored at 5°C until they were ready to load with the peptide active ingredients.

**Table 2**

| Sample Details | Polymer weight (g) | NMP weight (g) |
|---|---|---|
| 40%w/w of 75:25 PDLCL, 5.3 kDa, acid-initiated polymer in ∼60% w/w NMP | 20.02 | 30.06 |
| 40%w/w of 75:25 PDLCL, 4.3 kDa, dodecanol-initiated polymer in -60% w/w NMP | 20.11 | 30.17 |
| 40%w/w of 75:25 PDLCL, 6.6 kDa, PEG-300-initiated polymer in -60%w/w NMP (See Table 1, Sample No. 12) | 20.12 | 30.15 |
| Molar Ratio of (DL+CL/EG) = -2.1 | | |
| Molar % of DLCLEG = 49.3:18.2:32.6 | | |

*LPT Formulations.* LPT polymer-peptide formulations were prepared by adding a peptide and a polymer solution (i.e., a copolymer of the invention combined with solvent) into either a 20 mL or 40 mL vial, as appropriate, and by mixing the contents for 30 seconds with a vortex mixer. For each of the LPT polymer-peptide formulations described in these examples, the peptide was added to the formulation at between about 0.3% and 0.5% w/w. For each LPT polymer-peptide formulation, the individual samples were tested for stability at targeted storage times as outlined in the below Examples.

In the Examples described below, LPT polymer-peptide formulation were prepared comprising: (1) approximately 40% w/w of a 75:25 poly(DL-lactide-co-ε-caprolactone) (PDLCL) liquid polymer of the indicated molecular weight and produced using the indicated initiator; (2) approximately 60% w/w of N-methyl-2-pyrrolidone (NMP); and (3) between about 0.3% and about 0.5% of a peptide, as indicated.

To prepare LPT-peptide formulations comprising a divalent metal salt, a mixture of the peptide and corresponding metal salt solution was prepared and lyophilized. The lyophilized peptide/metal salt mixture was then combined with the LPT polymer solution. Briefly, first, the peptide was mixed with a concentration of metal salt solution in water. This solution was mixed well using a vortex or stir plate (based on quantities) until all peptide is dissolved well. By way of example, the preparation of Sample AA1 in Example 4 below involved preparing a solution of 1.3 mg/mL magnesium chloride in water followed by adding approximately 30 mg of abaloparatide acetate to 12 mL of 1.3 mg/mL magnesium chloride solution in a 20 mL scintillation vial and then vortexing until all peptide is dissolved in metal salt solution. Following this step, the mixture of peptide and metal salt solution was lyophilized by a freeze drying technique. Analysis of the lyophilized peptide/metal salt mixture (lyophilized cake) using reverse phase ultra high performance liquid chromatography (RP-UHPLC) was used for determining the peptide content and calculating the actual required quantity of lyophilized peptide/metal salt mixture to provide the desired quantity of peptide in the formulation (e.g., referring to Sample AA1 in Example 4, 5 mg of abaloparatide acetate per 1 g of final LPT-peptide formulation, *i.e.* 0.5%w/w peptide in the formulation)). Finally, the desired quantity of lypophilized peptide/metal salt was weighed into a 20 mL scintillation vial and is mixed with the appropriate quantity of LPT polymer solution. For example, approximately 7.5 mg of lyophilized peptide/metal salt (*e.g*., 2:1 peptide:metal salt ratio by weight as shown in Table 4 for Sample AA1) was added to approximately 992.5 mg polymer solution to form the LPT polymer solution.

### Example 2: LPT Formulations Comprising Abaloparatide Acetate and LPT Liquid Polymers Initiated with Different Polymer Initiators

This Example illustrates that the stability of abaloparatide is improved in LPT formulations comprising a PEG-initiated LPT polymer, as compared to LPT formulations comprising acid- or dodecanol-initiated LPT polymer.

Abaloparatide acetate (AA) is an exemplary peptide that has at least one accessible amine group according to the present invention. Specifically, AA has seven accessible amine groups in the form of four accessible lysine groups and three accessible arginine groups in the peptide, as well as an N-terminus. The LPT polymer-abaloparatide acetate formulations were prepared according to the procedure outlined in Example 1. In this Example, the LPT polymer in all cases was a copolymer of D,L-lactide and ε-caprolactone ("PDLCL") having a lactide-to-caprolactone molar ratio of about 75:25 or 25:75 as indicated, where the copolymer was initiated with the indicated initiator (see Table 3). In the case of the PEG300-initiated copolymer, the lactide-to-caprolactone-to-ethylene glycol ratio was about 57:21.3:21.7 (see Sample No. 9, Table 1). All formulations comprised NMP as the solvent in an amount that yielded about a 40:60 weight ratio of copolymer to solvent. In each formulation, about 5 mg abaloparatide acetate peptide was combined with about 1000 mg LPT polymer solution (*e.g*., peptide at approximately 0.5% w/w in the formulation).

For stability testing, the samples were incubated at 25°C in an oven, until the targeted storage time was reached and at that point, a single sample of a given LPT polymer-abaloparatide acetate formulation was removed from the incubator and tested for abaloparatide acetate content.

Prior to testing, the samples were allowed to sit to bring the sample temperature to room temperature and the vials were vortexed for about 60 seconds prior to extraction. For the sample analysis, 2.0 mL of 50:50 acetonitrile/methanol was carefully transferred to the scintillation vial and the cap was replaced, vortexed for 1 minute followed by sonication for 2 minutes. To the same scintillation vial, 15.0 mL of 65:35 water/methanol was added, the cap was replaced and vortexed again for 1 minute followed by sonication for 2 minutes. Samples were filtered through a 0.2 µm polytetrafluoroethylene (PTFE) filter into an high performance liquid chromatography (HPLC) vial after discarding the first ~1 mL aliquot. The samples were analyzed by reverse phase-ultra-high performance liquid chromatography (RP-UHPLC) using a standard peptide assay method. In later Examples described herein, dilutions were doubled for analyzing samples with peptides other than abaloparatide acetate in order to adjust the sample concentrations based on absorbance of the peptide; hence, the assay can accommodate peak intensity with the same method parameters.

The results of the stability tests are provided in Table 3 which shows (1) the molecular weight of the copolymer used in each of the formulations, (2) molar ratio of D,L-lactide (DL) to ε-caprolactone (CL) monomers, (3) the initiator used to form the liquid copolymer, and (4) the percent recovery (w/w) of abaloparatide acetate from the LPT-abaloparatide acetate formulation after a given number of days on stability (*i.e.*, in storage).

**Table 3**

| Formulation ID | AAX1 | AAX2 | AAX3 | AAX4 |
|---|---|---|---|---|
| Polymer MW (kDa) | 7.6 kDa | 5.2 kDa | 7.9 kDa | 18.2 kDa |
| Molar Ratio D,L-lactide (DL) to | 25:75 | 75:25 | 75:25 | 75:25 |
| ε-Caprolactone Monomers | | | | |
| Initiator | Glycolic Acid | | PEG300 | Dodecanol |
| | | | | |
| % Recovery @ 0 days | 94.1 | 98.6 | 94.0 | 104.6 |
| % Recovery @ 1 days | 49.9 | 46.5 | 88.1 | 69.9 |
| % Recovery @ 3 days | 7.1 | 0.8 | 53.5 | 16.5 |
| % Recovery @ 9 days | 0.0 | 0.0 | 19.3 | 0.0 |

Figure 1 shows the results of the stability tests for the various LPT polymer-abaloparatide acetate formulations at 25°C (AAX1-AAX4). The results show that the stability (measured by percentage w/w recovery of peptide from the formulation) of abaloparatide acetate in the PEG-300 initiated polymer (AAX3, ▲) was higher compared to formulations prepared using acid initiated polymers (AAX1, ◆ = 25:75 acid-initiated PDLCL; AAX2, ■ = 75:25 acid-initiated PDLCL) or dodecanol-initiated polymer (AAX4, •). For the PEG-300 initiated polymer, the percent recovery of abaloparatide was about 19% after 9 days at 25°C, whereas in the acid initiated and dodecanol initiated polymers essentially all of abaloparatide had already degraded. The two acid-initiated polymers, with varying ratios of D,L-lactide-to-ε-caprolactone monomers (75:25 or 25:75), display similar peptide recovery profiles.

These results show that abaloparatide can be stabilized in a PEG-initiated LPT polymer formulation, relative to the acid-initiated and dodecanol initiated LPT polymer formulations.

### Example 3: Mechanism of Peptide Degradation of LPT Formulations Comprising Peptides with Accessible Amine Groups

This Example illustrates that the peptide degradation observed in the LPT polymer formulations comprising abaloparatide acetate in Example 2 is due to acylation resulting in the addition of lactoyl fragments onto abaloparatide.

Liquid chromatography-mass spectrometry (LC-MS) and HPLC were used to identify the degradation products and mechanism of abaloparatide acetate in the presence of the acid-initiated and PEG-300 initiated LPT polymers (75:25 PDLCL) of Example 2. For each LPT polymer, data was collected on a freshly prepared sample (storage time of 0 days) and one that had been incubated at 25°C for 2 days in the case of the acid-initiated LPT polymer and 3 days in the case of the PEG-300 initiated polymer.

Figures 2A and 2B show an expanded portion of HPLC chromatograms for the acid initiated LPT polymer-abaloparatide acetate formulation at 0 and 2 days, respectively, at 25°C. A strong signal for abaloparatide acetate is observed at about 12.5 minutes. The chromatogram collected on the aged (2 day) formulation shows a degradant peak, eluted next to abaloparatide acetate, is a result of peptide acylation, which was also supported by LC-MS/MS results (data not shown). The addition of multiples of 144 Da were observed in LC-MS/MS chromatograms, leading to the hypothesis that acylation (+72 Da) was potentially occurring in some combination of the N-terminus, arginine, and/or lysine groups of abaloparatide. It was found that acylation was due to interaction with lactide monomer of PDLCL copolymer, resulting in addition of lactoyl fragments onto abaloparatide. This was found to be the main degradation mechanism occurring for the abaloparatide formulations. The 2 day old samples showed evidence of anywhere from 1 to 6 modifications of abaloparatide (primarily in even numbers) by lactide. The exact location of modifications occurring on abaloparatide was not studied in more detail.

A similar experiment was performed for PEG-300 initiated LPT polymer-abaloparatide acetate formulations at 0 and 3 days at 25°C. The analysis showed a similar acylation pattern of abaloparatide with additions of 72 Da corresponding to lactoyl fragments (data not shown). However, it was observed that the abundance of acylated species in the PEG300 initiated polymer sample was notably lower than that observed in the acid initiated polymer samples. This is consistent with the increased stability (percent recovery profile) observed for the PEG-300 initiated LPT polymer-abaloparatide acetate formulation at 25°C versus that observed for the acid-initiated formulation in Example 2 (see Table 3).

### Example 4: LPT Formulations Comprising Abaloparatide Acetate and Divalent Metal Salts

This Example illustrates that the stability of abaloparatide acetate is further improved in LPT formulations comprising PEG-initiated LPT polymer by addition of divalent metal ions.

LPT polymer-abaloparatide formulations with and without metal chloride salts were prepared according to the procedure outlined in Example 1. In this Example, the copolymer used for all of the formulations was a copolymer of PEG300-initiated PDLCL having a lactide-to-caprolactone molar ratio of about 75:25, and a lactide-caprolactone-ethylene glycol ratio of about 57:21.3:21.7, and an average molecular weight of 7.9 kDa (see, *e.g*., Table 1, Sample No. 9). The formulations comprised NMP as the solvent in an amount that yielded about a 40:60 weight ratio of polymer to solvent. To prepare LPT-peptide formulations comprising a divalent metal salt, a mixture of the abaloparatide acetate peptide and corresponding metal salt (see Table 4) was prepared as a solution and lyophilized. The lyophilized peptide/metal salt mixture was then combined with the LPT polymer solution at about 5 mg abaloparatide acetate peptide to about 1000 mg LPT polymer solution (*e.g*., approximately 0.5% w/w peptide in the formulation).

For accelerated stability testing, the samples were incubated at 37°C, until the targeted storage time was reached. The samples were analyzed for the abaloparatide acetate content recovered in %w/w according to the procedure outlined in Example 2.

The results of the stability tests are provided in Table 4 for formulations comprising metal salts, AA1 - AA3, and a control formulation that contains no metal salt, AAX8. Table 4 shows (1) metal salt used, (2) the concentration of the metal salt solution (mg/mL) in the final formulation, (3) the weight/weight ratio of abaloparatide acetate to metal salt in the final formulation (Peptide:Metal Salt Ratio) and (4) the percentage recovery (%w/w) of abaloparatide acetate from the LPT polymer-abaloparatide acetate formulation after a given number of days of storage at 37°C.

**Table 4**

| Formulation ID | AAX8 | AA1 | AA2 | AA3 |
|---|---|---|---|---|
| Metal salt | None | Magnesium Chloride | Calcium Chloride | Zinc Chloride |
| Salt conc. (mg/mL) | N/A | 1.25 | 0.8 | 0.38 |
| Peptide:Metal Salt Ratio | N/A | 2:1 | 6:1 | 13:1 |
| % Recovery @ 0 days | 92.1 | 96.6 | 95.6 | 94.6 |
| % Recovery @ 1 days | 77.1 | 97.6 | 99.1 | 90.7 |
| % Recovery @ 3 days | 13.0 | 92.3 | 89.2 | 73.1 |
| % Recovery @ 7 days | 0.0 | 88.4 | 73.6 | 46.1 |
| % Recovery @ 21 days | 0.0 | 81.9 | 22.2 | 0.0 |

Figure 3 shows the results of the recovery tests at 37°C for the various LPT polymer-abaloparatide acetate formulations comprising divalent metal chloride salts (AA1 = magnesium chloride, □; AA2 = calcium chloride, ■; AA3 = zinc chloride, •) versus a control formulation that contains no metal salt (AAX3 = no metal salt, ▲). The results show that the control formulation, AAX8 (▲), degraded faster than Formulations AA1, AA2 and AA3 and peptide (abaloparatide acetate) recovery after 3 days at 37°C was only 13%, and that almost all of the peptide was lost after 7 days at 37°C. All of the LPT polymer-abaloparatide acetate formulations comprising a metal chloride salt displayed enhanced stabilization, as compared to the formulation with no metal salt, resulting in a higher percent recovery of abaloparatide acetate over an extended period of time. The formulation that included magnesium chloride (□) showed the highest level of stability with about 82% abaloparatide acetate recovered after 21 days at 37°C, followed by the formulation that included calcium chloride (■) and then zinc chloride (•).

These results demonstrate that the stability of abaloparatide acetate in PEG-initiated LPT polymer formulations is further improved by inclusion of divalent metal salts.

### Example 5: LPT Formulations Comprising Abaloparatide Acetate and Magnesium Salts

This Example illustrates that the stability of abaloparatide is further improved in LPT formulations comprising a PEG-initiated LPT polymer by addition of magnesium ions.

The various LPT polymer-abaloparatide formulations with and without a metal salt were prepared according to the procedure outlined in Example 1. In this Example, the copolymer used for all of the formulations was a copolymer of PEG300-initiated PDLCL having a lactide-to-caprolactone molar ratio of about 75:25, and a lactide-caprolactone-ethylene glycol ratio of about 57:21.3:21.7, and an average molecular weight of 7.9 kDa (see, *e.g.*, Table 1, Sample No. 9). The formulations comprised NMP as the solvent in an amount that yielded about a 40:60 weight ratio of polymer to solvent. To prepare LPT-peptide formulations comprising a magnesium salt, a mixture of the abaloparatide acetate peptide and corresponding magnesium salt (see Table 5) was prepared and lyophilized. The lyophilized peptide/metal salt mixture was then combined with the LPT polymer solution at about 5 mg abaloparatide acetate peptide to about 1000 mg LPT polymer solution (e.g., approximately 0.5% w/w peptide in the formulation).

The samples were incubated at 37°C, until the targeted storage time was reached. The samples were analyzed for the percentage recovery of abaloparatide acetate according to the procedure outlined in Example 2.

The results of the stability tests are provided in Table 5A for formulations comprising metal salts, AA4 - AA7, and a control formulation that contains no metal salt, AAX9. Table 5A shows (1) metal salt used, (2) the concentration of the metal salt in the final formulation, (3) the weight ratio of abaloparatide acetate to metal salt in the final formulation (Peptide:Metal Salt Ratio) and (4) the recovery of abaloparatide acetate from the LPT peptide-abaloparatide acetate formulation after a given number of days of storage at 37°C.

**Table 5A**

| Formulation ID | AAX9 | AA4 | AA5 | AA6 | AA7 |
|---|---|---|---|---|---|
| Metal salt | None | Magnesium Acetate | | Magnesium Chloride | |
| Salt conc. (mg/mL) | N/A | 1.1 | 2.2 | 1.25 | 2.5 |
| Peptide:Metal Salt Ratio | N/A | 4.5:1 | 2.3:1 | 4:1 | 2:1 |
| % Recovery @ 0 days | 99.67 | 99.47 | 98.98 | 102.50 | 104.36 |
| % Recovery @ 1 days | 83.06 | 87.31 | 92.64 | 99.30 | 100.88 |
| % Recovery @ 3 days | 74.02 | 95.05 | 95.05 | 98.51 | 98.92 |
| % Recovery @ 7 days | 53.45 | 72.73 | 89.7 | 96.05 | 99.55 |
| % Recovery @ 10 days | 41.28 | 75.46 | 87.50 | 84.32 | 93.62 |
| % Recovery @ 22 days | 9.18 | 75.85 | 85.75 | 63.15 | 85.36 |

Figure 4 shows the results of the stability tests at 37°C for the various PEG-initiated LPT polymer-abaloparatide acetate formulations comprising magnesium salts versus a control formulation that contains no metal salt (AA4-AA7 and AAX9, respectively). The results show that the control formulation, AAX9 (▲), degraded over half of the abaloparatide acetate after 10 days of storage at 37°C, and only about 9% of the peptide was recovered from the Day 22 storage sample. The PEG-initiated LPT polymer-abaloparatide comprising the magnesium salts (both magnesium acetate (Mg(OAc)₂) and magnesium chloride (MgCl₂)) displayed enhanced stabilization at all concentrations tested, resulting in less degradation of abaloparatide acetate during storage (see Figure 4: AA6 = lower concentration MgCl₂ (x); AA7 = higher concentration MgCl₂ (□); AA4 = lower concentration Mg(OAc)₂ (∘); AA5 = higher concentration Mg(OAc)₂ (◇)). For both MgCl₂ and Mg(OAc)₂, a higher concentration of magnesium salts provided enhanced stabilization compared to lower concentrations of magnesium salts.

To further evaluate the effect of magnesium acetate (Mg(OAc)₂) on PEG-initiated LPT polymer-peptide formulations, a second experiment was performed. In this experiment, a PEG300-initiated, copolymer of PDLCL having a lactide-to-caprolactone molar ratio of about 75:25 was prepared, yielding a copolymer with a lactide-to-caprolactone-to-ethylene glycol molar ratio of 57:21.3:21.7, and an average molecular weight of 7.9 kDa (see Sample No. 9, Table 1). The formulations comprised NMP as the solvent in an amount that yielded about a 40:60 weight ratio of polymer to solvent. To prepare LPT-peptide formulations comprising magnesium acetate, a mixture of the abaloparatide acetate peptide and magnesium acetate was prepared and lyophilized. The lyophilized peptide/metal salt mixture was then combined with the LPT polymer solution at about 5 mg abaloparatide acetate peptide to about 1000 mg LPT polymer solution (e.g., approximately 0.5% w/w peptide in the formulation).

The samples were incubated at 37°C, until the targeted storage time was reached. The samples were analyzed for the abaloparatide acetate according to the procedure outlined in Example 2.

The results of the stability tests are provided in Table 5B for formulations comprising metal salts, AA8 and AA9, and a control formulation that contains no metal salt, AAX10. Table 5B shows (1) metal salt used, (2) the concentration of the metal salt in the final formulation, (3) the weight ratio of peptide to metal salt in the final formulation; and (4) the percentage recovery of abaloparatide acetate from the LPT peptide-abaloparatide acetate formulation after a given number of days of storage at 37°C.

**Table 5B**

| Formulation ID | AAX10 | AA8 | AA9 |
|---|---|---|---|
| Metal salt | None | Magnesium Acetate | |
| Salt conc. (mg/mL) | N/A | 0.55 | 1.1 |
| Peptide:Metal Salt Ratio | N/A | 9:1 | 4.5:1 |
| % Recovery @ 3 days | 30.82 | 61.22 | 94.13 |
| % Recovery @ 7 days | 13.81 | 26.51 | 85.68 |
| % Recovery @ 14 days | 1.76 | 9.99 | 80.28 |
| % Recovery @ 21 days | 0.80 | 13.63 | 83.52 |
| % Recovery @ 30 days | 0.00 | 4.42 | 82.84 |
| % Recovery @ 60 days | 0.00 | 0.00 | 78.17 |

Figure 5 shows the result of the stability testing of test formulations AA8 (∘) and AA9 (0) relative to the control formulation, AAX10 (▲).

Figure 5 shows that the control formulations AAX10 (▲) degraded faster than Formulations AA8 and AA9, and recovery of the abaloparatide acetate after three days of storage at 37°C was only 30.82%, and that almost all of the peptide was degraded after 14 days of storage at 37°C. The PEG-initiated LPT polymer-abaloparatide acetate formulations comprising magnesium acetate as the metal salt exhibited slower peptide degradation profiles relative to the control formulation. The addition of lower concentrations of magnesium acetate (0.55 mg/mL, AA8) (∘) resulted in a higher recovery profile compared to the control formulation, with the largest differences at the shorter storage time of 3 days. The addition of higher concentrations of magnesium acetate (1.1 mg/mL, AA9) (0) resulted in a significantly higher recovery profile over the entire 60 day storage period. Notably, after 60 days of storage at 37°C about 78% of the peptide was recovered from the formulation. These results indicate that abaloparatide acetate can be stabilized for extended storage by addition of magnesium acetate, particularly when the magnesium acetate is added in higher concentrations.

Taken together, these results demonstrate that the stability of abaloparatide acetate in PEG-initiated LPT formulations is improved by inclusion of magnesium salts at suitable amounts.

### Example 6: LPT Formulations Comprising Abaloparatide Acetate and Zinc Salts

This Example illustrates that the stability of abaloparatide is further improved in LPT formulations comprising a PEG-initiated LPT polymer by addition of zinc ions.

The various LPT polymer-abaloparatide acetate formulations with and without a metal salt were prepared according to the procedure outlined in Example 1. In this experiment, a PEG300-initiated, copolymer of PDLCL having a lactide-to-caprolactone molar ratio of about 75:25 was prepared, yielding a copolymer with a lactide-to-caprolactone-to-ethylene glycol molar ratio of 57:21.3:21.7, and an average molecular weight of 7.9 kDa (see Sample No. 9, Table 1). The formulations comprised NMP as the solvent in an amount that yielded about a 40:60 weight ratio of polymer to solvent. To prepare LPT-peptide formulations comprising zinc salts (ZnCl₂ or Zn(OAc)₂), a mixture of the abaloparatide acetate peptide and zinc salt was prepared and lyophilized. The lyophilized peptide/metal salt mixture was then combined with the LPT polymer solution at about 5 mg abaloparatide acetate peptide to about 1000 mg LPT polymer solution (*e.g*., approximately 0.5% w/w peptide in the formulation).

The samples were incubated at 37°C, until the targeted storage time was reached. The samples were analyzed for the abaloparatide acetate according to the procedure outlined in Example 2.

Table 6 shows (1) the metal salt used in each of the test formulations, (2) the concentration of the metal salt in the final formulation, (3) the weight ratio of peptide to metal salt in the final formulation (Peptide:Metal Salt Ratio); and (4) the recovery of abaloparatide acetate as a function of days of storage at 37°C for test formulations AA10-AA12 and also for the control formulation AAX11, which did not comprise a metal salt.

**Table 6**

| Formulation ID | AAX11 | AA10 | AA11 | AA12 |
|---|---|---|---|---|
| Metal salt | None | Zinc Acetate | | Zinc Chloride |
| Salt conc. (mg/mL) | N/A | 2.5 | 5.68 | 1.0 |
| Peptide:Metal Salt Ratio | N/A | 2:1 | 0.88:1 | 5:1 |
| % Recovery @ 3 days | 30.82 | 96.75 | 86.84 | 45.51 |
| % Recovery @ 7 days | 13.81 | 90.66 | 57.99 | 9.98 |
| % Recovery @ 14 days | 1.76 | 69.66 | 28.42 | 0.00 |
| % Recovery @ 21 days | 0.80 | 60.98 | 21.30 | 0.00 |

Figure 6 shows the result of the stability testing of test formulations AA10-AA12 relative to the control formulation, AAX11. Figure 6 shows that the PEG-initiated LPT polymer-abaloparatide acetate formulations comprising zinc acetate as the metal salt also exhibited higher recovery profiles relative to the control formulation (▲). The LPT polymer-abaloparatide acetate formulation comprising lower concentrations of zinc acetate (2.5 mg/mL, AA10) ( 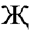 ) resulted in a higher recovery of abaloparatide compared to the formulation comprising higher concentrations of zinc acetate (5.68 mg/mL, AA11) (+) for a given period of time, with about 40% versus 80% recovery of the peptide after 21 days of storage at 37°C. Figure 6 also shows that LPT polymer-abaloparatide acetate formulations comprising zinc chloride (1 mg/mL, AA12) (◆) displayed a higher recovery at 3 days of storage, but in this experiment, did not display enhanced stabilization of the peptide relative to the control formulation at storage times of about 7 days and beyond.

These results indicate that the stability of abaloparatide acetate in PEG-initiated LPT polymer formulations can be improved by inclusion of zinc salts, and particularly, zinc acetate.

### Example 7: LPT Formulations Comprising Leuprolide Acetate and LPT Liquid Polymers Initiated with Different Polymer Initiators

This Example illustrates that the stability of leuprolide acetate is greater in LPT formulations comprising an acid initiated LPT polymer, compared to a PEG initiated or dodecanol initiated polymer.

Leuprolide acetate is a linear peptide exemplifying a peptide with few, one or no accessible amine groups. Specifically, leuprolide acetate is a linear nonapeptide containing one arginine group in its structure, *i*.*e*., it possesses one amine group, although, as discussed below, the amine group is not *accessible* according to the invention due to steric hindrance caused by a bulky isobutyl group of D-leucine. The various LPT polymer-leuprolide acetate formulations were prepared according to the procedure outlined in Example 1. In this Example, the LPT polymer in all cases was a copolymer of D,L-lactide and ε-caprolactone ("PDLCL") having a lactide-to-caprolactone molar ratio of about 75:25, where the copolymer was initiated with the indicated initiator (see Table 3). In the case of the PEG300-initiated copolymer, the lactide-to-caprolactone-to-ethylene glycol ratio was about 49.3:18.2:32.6 (see Sample No. 12, Table 1). All formulations comprised NMP as the solvent in an amount that yielded about a 40:60 weight ratio of copolymer to solvent. In each formulation, about 4 mg leuprolide acetate peptide was combined with about 1000 mg LPT polymer solution (*e.g*., approximately 0.4% w/w peptide in the formulation).

The samples were incubated at 37°C, until the targeted storage time was reached. The samples were analyzed for the amount of leuprolide acetate according to the procedure outlined in Example 2.

The results of the stability tests are provided in Table 7 which shows (1) the weight average molecular weight of the copolymer used in each of the formulations, (2) the initiator used to form the liquid copolymer, and (3) the recovery of leuprolide acetate from the LPT polymer-leuprolide acetate formulation after a given number of days of storage at 37°C.

**Table 7**

| Formulation ID | LAX 1 | LAX2 | LAX3 |
|---|---|---|---|
| Polymer MW (kDa) | 5.3 kDa | 6.6 kDa | 4.3 kDa |
| Initiator | Glycolic Acid | PEG300 | Dodecanol |
| % Recovery @ 0 days | 102.73 | 97.76 | 98.72 |
| % Recovery @ 3 days | 85.30 | 26.14 | 25.66 |
| % Recovery @ 7 days | 83.46 | 23.48 | 19.62 |
| % Recovery @ 14 days | 62.49 | 20.48 | 18.15 |
| % Recovery @ 30 days | 41.31 | 19.05 | 16.00 |

Figure 7 shows recovery profiles for LPT polymer-leuprolide acetate formulations produced with the three initiators (LAX1-LAX3) at 37°C. The results show that the stability of leuprolide acetate in the acid initiated polymer (■) was substantially higher compared to formulations prepared using either PEG-300 initiated polymers (▲) or dodecanol initiated polymer (•). For the acid initiated polymer, the percent recovery of leuprolide acetate was about 41% after 30 days at 37 °C, whereas in the PEG-300 initiated and dodecanol initiated polymers the recovery at 30 days was approximately 19% and 16%, respectively. The results show that acid-initiated polymers provide an improved stability profile for leuprolide acetate formulations compared to dodecanol initiated or PEG initiated LPT polymers.

As discussed above, leuprolide acetate is a linear peptide containing one arginine group in its structure, *i.e.*, containing one amine group. Although, arginine is more basic than lysine due to its resonance structures which stabilize/delocalize the positive charge on arginine by donating electron pair from other two nitrogens on the sidechain, leuprolide acetate was nonetheless stable in the acid-initiated LPT formulations, for example, as compared to abaloparatide acetate, which was not stable in the acid-initiated LPT formulations. This unexpected higher stability of leuprolide acetate can be explained by steric hindrance caused by the bulky isobutyl group of D-leucine which protects the guanidinium group of arginine from acidic environment and slows down the reaction with polymer.

### Example 8: LPT Formulations Comprising Leuprolide Acetate and Metal Salts

This Example illustrates that the stability of leuprolide acetate is further improved in LPT formulations comprising an acid initiated polymer by the addition of divalent metal ions.

The various LPT polymer-leuprolide acetate formulations were prepared according to the procedure outlined in Example 1. In this Example, the LPT polymer in all cases was a copolymer of D,L-lactide and ε-caprolactone ("PDLCL") having a lactide-to-caprolactone molar ratio of about 75:25, where the copolymer was initiated with glycolic acid, yielding a copolymer with a weight average molecular weight of 5.3 kDa. All formulations comprised NMP as the solvent in an amount that yielded about a 40:60 weight ratio of copolymer to solvent. To prepare LPT-peptide formulations comprising metal salts, a mixture of the leuprolide acetate peptide and metal salt indicated in Table 8 was prepared and lyophilized. The lyophilized peptide/metal salt mixture was then combined with the LPT polymer solution at about 4 mg leuprolide acetate peptide to about 1000 mg LPT polymer solution (*e.g*., approximately 0.4% w/w peptide in the formulation).

The samples shown in Table 8 were incubated at 37°C, until the targeted storage time was reached. The samples were analyzed for the leuprolide acetate according to the procedure outlined in Example 2.

Table 8 shows (1) the metal salt used in each of the test formulations, (2) the concentration of the salt in the final formulation, (3) the weight ratio of peptide to metal salt in the final formulation (Peptide:Metal Salt Ratio) and (4) the percentage recovery of leuprolide acetate (w/w) after the indicated number of days of storage days of storage at 37°C.

**Table 8**

| Formulation ID | LAX4 | LA1 | LA2 | LA3 | LA4 | LA5 | LA6 |
|---|---|---|---|---|---|---|---|
| Metal salt | None | Magnesium acetate | | Zinc acetate | | Zinc chloride | |
| Salt conc. (mg/mL) | N/A | 0.53 | 1.08 | 2.14 | 5.0 | 0.15 | 0.93 |
| Peptide:Metal Salt Ratio | N/A | 7.5:1 | 3.7:1 | 1.87:1 | 0.8:1 | 26:1 | 4.3:1 |
| % Recovery @ 0 days | 104.86 | 103.95 | 101.18 | 98.08 | 98.38 | 99.87 | 99.02 |
| % Recovery @ 3 days | 85.06 | 85.57 | 82.66 | 89.72 | 91.49 | 89.28 | 93.29 |
| % Recovery @ 7 days | 34.61 | 35.29 | 64.70 | 81.89 | 74.94 | 78.37 | 83.52 |
| % Recovery @ 14 days | 26.61 | 27.14 | 50.76 | 61.33 | 57.46 | 58.32 | 69.98 |
| % Recovery @ 21 days | 21.56 | 22.52 | 42.25 | 53.04 | 48.62 | 49.99 | 60.90 |
| % Recovery @ 30 days | 18.20 | 19.78 | 35.66 | N/A | N/A | N/A | N/A |
| % Recovery @ 60 days | 13.54 | 13.27 | 22.13 | N/A | N/A | N/A | N/A |

Figure 8 shows recovery profiles for acid-initiated leuprolide acetate formulations comprising, a higher concentration of magnesium acetate (1.08 mg/mL, LA2) (0) and a lower concentration of magnesium acetate (0.53 mg/mL, LA1) (∘) compared to the control formulation that did not include a metal salt (LAX4) (■) over a storage period of up to 60 days at 37°C. The recovery profiles for the three formulations are similar at storage times of 3 days and less. At storage times of 7 days and longer, the formulation comprising a higher concentration of magnesium acetate (1.08 mg/mL, LA2) show improved stability, resulting in a higher recovery of leuprolide acetate compared to the control formulation as well as the formulation comprising a lower concentration of magnesium acetate (0.53 mg/mL, LA1). The recovery profile for the formulation comprising a lower concentration of magnesium acetate is similar to the recovery profile for the control formulation. These results indicate that the leuprolide acetate can be stabilized by the combination of the acid initiated LPT polymer and magnesium acetate.

Figure 9 shows recovery profiles for acid-initiated LPT polymer-leuprolide acetate formulations comprising either zinc acetate (2.14 mg/mL, LA3 and 5.0 mg/mL, LA4) (( 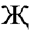 ) and (+), respectively) or zinc chloride (0.15 mg/mL, LA5 and 0.93 mg/mL, LA6) ((•) and (◆), respectively) as the metal salts, compared to the acid-initiated control formulation that did not include a metal salt (LAX4) (■), over a storage period of up to 21 days at 37°C. The leuprolide acetate in the formulations comprising either zinc chloride (LA5 and LA6) or zinc acetate (LA3 and LA4) are slower to degrade when compared to the control formulation, with a significant difference in the recovery profiles of leuprolide acetate for formulations comprising either zinc chloride or zinc acetate on days 7, 14 and 21 compared to the control formulation. The formulation comprising higher concentration of zinc chloride as the metal salt (0.93 mg/mL, LA6) (◆) showed the highest recovery, especially at a longer storage time of 14 days and more.

These results indicate that the stability of leuprolide acetate in acid-initiated LPT polymer formulations can be improved by inclusion of zinc salts, including at lower and higher concentrations of such salts.

### Example 9: LPT Formulations Comprising Lanreotide Acetate and LPT Liquid Polymers Initiated with Different Polymer Initiators and Metal Salts.

This Example illustrates that the stability of lanreotide acetate is improved in LPT formulations comprising an acid initiated LPT Polymer and that the stability of lanreotide acetate can be further improved by addition of divalent metal ions such as magnesium or zinc.

LPT formulations comprising lanreotide acetate were produced as described in Example 1 and are shown in Table 9A and Table 9B. Lanreotide acetate is an exemplary cyclic peptide containing few, one or no accessible amine groups. Specifically, lanreotide acetate is a cyclic peptide containing one lysine group in its structure, *i.e.*, it possesses one accessible amine group, according to the invention. The various LPT polymer-lanreotide acetate formulations were prepared according to the procedure outlined in Example 1.

In this Example, the LPT polymer in all cases was a copolymer of D,L-lactide and ε-caprolactone ("PDLCL") having a lactide-to-caprolactone molar ratio of about 75:25. In the experiment described in Table 9A, the copolymer was initiated with the indicated initiator, yielding a copolymer having the indicated weight average molecular weight. In the case of the PEG300-initiated copolymer in Table 9A, the lactide-to-caprolactone-to-ethylene glycol ratio was about 49.3:18.2:32.6 (see Sample No. 12, Table 1). In the experiment shown in Table 9B, the LPT polymer used in all test formulations was a glycolic acid-initiated, 75:25 PDLCL having a weight average molecular weight of 5.3 kDa. All formulations comprised NMP as the solvent in an amount that yielded about a 40:60 weight ratio of copolymer to solvent. In each LPT formulation shown in Table 9A, about 3 mg lanreotide acetate peptide was combined with about 1000 mg LPT polymer solution (*e.g*., approximately 0.3% w/w peptide in the formulation). To prepare LPT-peptide formulations comprising metal salts shown in Figure 9B, a mixture of the lanreotide acetate peptide and metal salt indicated in Table 9B was prepared and lyophilized. The lyophilized peptide/metal salt mixture was then combined with the LPT polymer solution at about 3 mg lanreotide acetate peptide to about 1000 mg LPT polymer solution (*e.g*., approximately 0.3% w/w peptide in the formulation).

The samples were incubated at 37°C, until the targeted storage time was reached. The samples were analyzed for the amount of lanreotide acetate according to the procedure outlined in Example 2.

Table 9A shows (1) the weight average molecular weight of the copolymer used in each of the formulations, (2) the initiator used to form the liquid copolymer, (3) and the recovery of lanreotide acetate after 0, 3 and 7 days of storage at 37°C.

**Table 9A**

| Formulation ID | LNAX1 | LNAX2 | LNAX3 |
|---|---|---|---|
| Polymer MW (kDa) | 5.3 | 4.3 | 6.6 |
| Initiator | Acid | Dodecanol | PEG300 |
| % Recovery @ 0 days | 104.83 | 93.74 | 86.77 |
| % Recovery @ 3 days | 48.56 | 28.84 | 12.94 |
| % Recovery @ 7 days | 28.70 | 6.43 | 0.00 |

Figure 10 shows the result of the stability testing of the three formulations LNAX1 (acid-initiated, (■)) LNAX2 (dodecanol-initiated, (•)), and LNAX3 (PEG-initiated, (▲)). Figure 10 shows that in all three formulations LNAX1-LNAX3, which did not comprise metal salts, recovery of the peptide (lanreotide acetate) was substantially reduced after three days of storage at 37°C. In particular, in formulations in which the polymer was initiated with dodecanol (LNAX2) (•) or PEG300 (LNAX3) (▲), substantially all of the polymer was lost after seven days of storage at 37°C. Formulations comprising an acid-initiated copolymer (LNAX1) (■) displayed greater stability, with about 30% of the lanreotide acetate being recovered after seven days of accelerated stability testing at 37°C. Therefore, with the cyclic peptide having only one accessible amine group, the acid-initiated LPT polymer showed the greatest stability, as compared to dodecanol-initiated or PEG-initiated polymers.

Table 9B shows the results of additional experiments in which metal salts were added to the acid-initiated LPT formulation to determine whether the metal salts could improve stability of the peptide. Table 9B shows: (1) the metal salt used in each of the test formulations, (2) the concentration of the salt in the final formulation, (3) the weight ratio of lanreotide acetate to metal salt in the final formulation (Peptide:Metal Salt Ratio), (4) and the recovery of lanreotide acetate after 0, 3, 7 and 14 days of storage at 37°C, and in the case of zinc acetate and zinc chloride, also after 21 days of storage at 37°C.

**Table 9B**

| Formulation ID | LNAX4 | LNA1 | LNA2 | LNA3 | LNA4 | LNA5 | LNA6 |
|---|---|---|---|---|---|---|---|
| Metal salt | None | Magnesium acetate | | Zinc acetate | | Zinc chloride | |
| Salt conc. (mg/mL) | N/A | 0.55 | 1.1 | 2.3 | 5.0 | 0.2 | 1.0 |
| Peptide:Metal Salt Ratio | N/A | 5.5:1 | 2.7:1 | 1.3:1 | 0.6:1 | 15:1 | 3:1 |
| Recovery @ 0 days (%) | 101.33 | 114.15 | 102.81 | 103.18 | 103.51 | 101.32 | 104.03 |
| Recovery @ 3 days (%) | 46.68 | 20.79 | 37.15 | 65.80 | 66.00 | 63.20 | 80.04 |
| Recovery @ 7 days (%) | 18.40 | 7.25 | 12.09 | 59.74 | 56.24 | 54.34 | 89.30 |
| Recovery @ 14 days (%) | 5.76 | 1.60 | 1.51 | 22.72 | 15.55 | 25.72 | 54.92 |
| Recovery @ 21 days (%) | - | - | - | 9.61 | 7.94 | 14.48 | 44.32 |

Figure 11 shows the result of the stability testing of test formulations LNA1 (•) and LNA2 (◆), which add two different concentrations of magnesium acetate to the formuilations, relative to control formulation LNAX4 (■), which is a glycolic acid-initiated, 75:25 PDLCL having a weight average molecular weight of 5.3 kDa. Figure 11 shows that acid-intiated LPT polymer formulations comprising lanreotide acetate pharmaceutical formulations comprising both a lower concentration (0.55 mg/mL, LNA1) (•) and a higher concentration (1.1 mg/mL, LNA2) (◆) of magnesium acetate exhibited decreased or similar stability of the peptide relative to the acid-initiated control formulation that did not include a metal salt (LNAX1) (■) at both three days and seven days, and after fourteen days substantially no peptide was recovered from the magnesium acetate-containing, acid-initiated LPT formulations.

Figure 12 shows the results of the accelerated stability testing of test formulations LNA3 ( 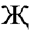 ), LNA4 (+), LNA5 (•) and LNA6 (◆), which add different concentrations of zinc acetate or zinc chloride, relative to control formulation LNAX4 (■). Figure 12 shows that acid-initiated LPT polymer formulations comprising lanreotide acetate and zinc salts are slower to degrade at 37°C than the control formulation that contains no metal salt. In Figure 12, the stability profiles of formulations comprising lower (2.3 mg/mL, LNA3) ( 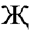 ) and higher (5.0 mg/mL, LNA4) (+) concentrations of zinc acetate and lower (0.2 mg/mL, LNA5) (•) and higher (1.0 mg/mL, LNA6) (◆) concentrations of zinc chloride are presented relative to the percent recovery of the acid-initiated control formulation (LNAX4) (■). As shown in Figure 12, all four of the test formulations containing a zinc salt exhibit improved stability over at least about 21 days of storage at 37°C, with the improvement being particularly marked at about seven days. In particular, while formulations comprising zinc acetate and lower concentrations of zinc chloride all performed equally well, the formulation comprising the higher concentration of zinc chloride (LNA6, ◆) provided significantly enhanced stability of the peptide even relative to the other test formulations across the entire length of the 21-day study.

These results indicate that the stability of lanreotide acetate in liquid polymer pharmaceutical formulations can be improved by inclusion of zinc salts. Particularly, relatively high concentrations (about 1.0 mg/mL) of zinc chloride appear to allow at least about 40% of the peptide to be recovered after 21 days of storage at 37°C. Notably, zinc acetate appeared to provide the best stabilizing effects at the lower concentration, while zinc chloride appeared to provide the best stabilizing effects at the higher concentration.

### Example 10: LPT Formulations Comprising Octreotide Acetate and LPT Liquid Polymers Initiated with Different Polymer Initiators and Metal Salts

This Example illustrates the stability of octreotide acetate in LPT formulations comprising LPT polymers synthesized using different initiators and divalent metal ions.

Octreotide acetate is another exemplary cyclic peptide containing few, one or no accessible amine groups. Specifically, octreotide acetate is a cyclic peptide containing one lysine group in its structure, *i.e.,* it possesses one accessible amine group, according to the invention. The various LPT polymer-octreotide acetate formulations were prepared according to the procedure outlined in Example 1. In this Example, the LPT polymer in all cases was a copolymer of D,L-lactide and ε-caprolactone ("PDLCL") having a lactide-to-caprolactone molar ratio of about 75:25. In the experiment described in Table 10A, the copolymer was initiated with the indicated initiator, yielding a copolymer having the indicated weight average molecular weight. In the case of the PEG300-initiated copolymer in Table 10A, the lactide-to-caprolactone-to-ethylene glycol ratio was about 49.3:18.2:32.6 (see Sample No. 12, Table 1). In the experiment shown in Table 10B, the LPT polymer used in all test formulations was a glycolic acid-initiated, 75:25 PDLCL having a weight average molecular weight of 5.3 kDa. All formulations comprised NMP as the solvent in an amount that yielded about a 40:60 weight ratio of copolymer to solvent. In each LPT formulation shown in Table 10A, about 3 mg octreotide acetate peptide was combined with about 1000 mg LPT polymer solution (e.g., approximately 0.3% peptide w/w of the formulation). To prepare LPT-peptide formulations comprising metal salts shown in Figure 10B, a mixture of the octreotide acetate peptide and metal salt indicated in Table 10B was prepared and lyophilized. The lyophilized peptide/metal salt mixture was then combined with the LPT polymer solution at about 3 mg octreotide acetate peptide to about 1000 mg LPT polymer solution (*e.g.,* approximately 0.3% w/w peptide in the formulation).

The samples were incubated at 37°C, until the targeted storage time was reached. The samples were analyzed for the amount of octreotide acetate according to the procedure outlined in Example 2.

Table 10A shows (1) the molecular weight of the copolymer used in each of the formulations, (2) the initiator used to form the liquid copolymer, and (3) the recovery of octreotide acetate as a function of storage time at 37°C.

**Table 10A**

| Formulation ID | OAX1 | OAX3 | OAX4 |
|---|---|---|---|
| Polymer MW (kDa) | 5.3 | 4.3 | 6.6 |
| Initiator | Glycolic Acid | Dodecanol | PEG300 |
| Recovery @ 3 days (%) | 24.74 | 20.52 | 7.99 |
| Recovery @ 7 days (%) | 7.74 | 3.48 | 0.00 |

Figure 13 shows the result of the stability tests for the LPT polymer-octreotide acetate formulations where the polymer was initiated with different polymer initiators as indicated in Table 10A (OAX1 = acid initiated (■), OAX3 = dodecanol initiated (•), and OAX4 = PEG300 initiated (A)). Figure 13 shows that all three LPT polymer-octreotide acetate formulations (OAX1, OAX3 and OAX4) show degradation of the octreotide acetate after storage at 37°C, and that acid-initiated LPT polymers (■) provide a slightly improved percent recovery compared to dodecanol-initiated (•) or PEG-initiated (▲) polymers.

Table 10B shows the results of additional experiments in which magnesium acetate was added to the acid-initiated LPT formulation to determine whether this metal salt could improve stability of the peptide. Table 10B shows: (1) the concentration of the magnesium acetate in the final formulation, (2) the weight ratio of peptide to metal salt in the final formulation (Peptide:Metal Salt Ratio), and (3) the recovery of octreotide acetate after 0, 3, 7 and 14 days of storage at 37°C.

**Table 10B**

| Formulation ID | OAX2 | OA1 | OA2 |
|---|---|---|---|
| Salt conc. (mg/mL) | None | 0.55 | 1.1 |
| Peptide:Metal Salt Ratio | N/A | 5.5:1 | 2.7:1 |
| Recovery @ 3 days (%) | 25.42 | 19.77 | 13.19 |
| Recovery @ 7 days (%) | 7.17 | 4.21 | 1.13 |
| Recovery @ 14 days (%) | 0.82 | 1.02 | 0.19 |

Figure 14 shows the result of the stability testing of acid initiated LPT polymer-octreotide acetate formulations comprising magnesium acetate, OA1 (∘) and OA2 (0), relative to control formulation OAX2 (■), which is a glycolic acid-initiated, 75:25 PDLCL having a weight average molecular weight of 5.3 kDa that does not comprise magnesium acetate. Figure 14 shows that acid-initiated LPT polymer-octreotide acetate formulations comprising magnesium acetate as the metal salt are characterized by rapid degradation of the peptide at 37°C. Both of the formulations comprising magnesium acetate (lower concentration = OA1 (∘) and higher concentration = OA2 (0)) exhibited decreased stability of the peptide relative to the acid-initiated control formulation that did not include a metal salt (OAX2) (■) at both three days and seven days, and after fourteen days substantially no peptide was recovered from all three formulations, including the control formulation.

Taken together with the results shown in the other Examples, while cyclic peptides such as lanreotide acetate and octreotide acetate were expected to degrade slower compared to linear peptides such as leuprolide acetate, surprisingly, leuprolide acetate showed higher stability in an acid initiated LPT formulation than lanreotide and octreotide. This may be mainly due to the steric hindrance effect which slows down the acylation (degradation) of leuprolide acetate. In addition, abaloparatide acetate, which like leuprolide acetate has a simple linear sequence, but additionally has a high number of accessible amine groups (i.e., active reaction sites), likely attracted the acid end group of acid-initiated PDLCL polymer, which resulted in high degradation in presence of such acid initiated polymer. In contrast, with leuprolide acetate, lanreotide acetate and octreotide acetate, having only one active reaction site, hydrophilicity appeared to play a dominant role in degradation of peptide and hence, those peptides were degraded more rapidly in PEG-initiated polymer, where surprisingly, abaloparatide acetate was notably more stable. Finally, the addition of divalent metal salts improved stability for many of the LPT formulations, with magnesium salts and zinc acetate being particularly effective for use with PEG-initiated LPT polymers and peptides with larger numbers of accessible amine groups, with zinc salts being particularly effective for acid-initiated polymers and fewer or no accessible amine groups.

Various modifications of the above described invention will be evident to those skilled in the art. It is intended that such modifications are included within the scope of the following claims.

## Claims

1. A pharmaceutical composition, comprising:
a) an active pharmaceutical ingredient comprising a peptide or a pharmaceutically acceptable ester or salt thereof, wherein the peptide comprises at least one accessible amine group; and
b) a biodegradable liquid block copolymer comprising:
i) a copolymer block comprising: (1) monomer residues selected from the group consisting of D,L-lactide, D-lactide, L-lactide, and glycolide, and combinations thereof and (2) monomer residues selected from the group consisting of ε-caprolactone, trimethylene carbonate, and combinations thereof; and
ii) a polymer block comprising a low-molecular weight polyethylene glycol (PEG);
wherein the biodegradable liquid block copolymer is not a reverse thermal gel and is synthesized by initiation with the low-molecular weight PEG; and
wherein a viscosity of the biodegradable liquid block copolymer does not spontaneously increase with an increase in temperature; and
wherein the end groups of the biodegradable liquid block copolymer are not covalently modified.

2. The pharmaceutical composition of claim 1, wherein the PEG is methoxy-PEG.

3. The pharmaceutical composition of any one of claims 1 and 2, wherein the copolymer block of (i) has a molar ratio of lactide or glycolide monomer residues to caprolactone and/or trimethylene carbonate monomer residues between about 10:90 and about 90:10.

4. The pharmaceutical composition of any one of claims 1 and 2, wherein the copolymer block of (i) has a molar ratio of lactide or glycolide monomer residues to caprolactone and/or trimethylene carbonate monomer residues between about 25:75 and about 75:25.

5. The pharmaceutical composition of any one of claims 1 and 2, wherein the copolymer block of (i) has a molar ratio of lactide monomer residues to caprolactone and/or trimethylene carbonate monomer residues of 75:25.

6. The pharmaceutical composition of any one of claims 1 to 5, wherein a molar ratio of ethylene glycol residues in the low-molecular weight PEG to all other monomer residues in the biodegradable copolymer is between about 10:90 and about 50:50.

7. The pharmaceutical composition of any one of claims 1 to 5, wherein a molar ratio of lactide or glycolide monomer residues to caprolactone and/or trimethylene carbonate monomers to ethylene glycol residues in the biodegradable copolymer is X:Y:Z, where X can be any number between about 25 and about 75, Y can be any number between about 5 and about 45, and Z can be any number between about 5 and about 55, such that the sum of X, Y, and Z is 100.

8. The pharmaceutical composition of any one of claims 1 to 7, wherein the polyethylene glycol has a number average molecular weight of 200 to 2000 daltons.

9. The pharmaceutical composition of any one of claims 1 to 8, further comprising a biocompatible solvent or combination or mixture of solvents and/or cosolvents.

10. The pharmaceutical composition of claim any one of claims 1 to 9, further comprising a divalent cation, wherein the divalent cation is provided as a metal salt or is selected from the group consisting of magnesium, calcium, and zinc.

11. The pharmaceutical composition of claim 10, wherein the metal salt is selected from the group consisting of magnesium acetate, magnesium chloride, calcium chloride, zinc chloride, and zinc acetate.

12. The pharmaceutical composition of claim 10 or 11, wherein the metal salt is magnesium acetate, and wherein the magnesium acetate is at a concentration of between about 0.01 mg/mL and about 2.75 mg/mL of the pharmaceutical composition; or
wherein the metal salt is magnesium chloride, and wherein the magnesium chloride is at a concentration of between about 0.01 mg/mL and about 3.75 mg/mL of the pharmaceutical composition; or
wherein the metal salt is calcium chloride, and wherein the calcium chloride is at a concentration of between about 0.01 mg/mL and about 1.6 mg/mL of the pharmaceutical composition; or
wherein the metal salt is zinc acetate, and wherein the zinc acetate is at a concentration of between about 0.01 mg/mL and about 8.2 mg/mL of the pharmaceutical composition; or
wherein the metal salt is zinc chloride, and wherein the zinc chloride is at a concentration of between about 0.01 mg/mL and about 1.4 mg/mL of the pharmaceutical composition.

13. The pharmaceutical composition of any one of claims 1 to 12, wherein the peptide comprises at least two basic amino acids selected from the group consisting of arginine, histidine, lysine, and combinations thereof.

14. The pharmaceutical composition of any one of claims 1 to 13, wherein a weight-average molecular weight of the biodegradable copolymer is between about 1 kDa and about 35 kDa.

15. The pharmaceutical composition of claim 9, wherein the biocompatible solvent comprises N-methyl-2-pyrrolidone (NMP).

16. The pharmaceutical composition of claim 9, wherein, after administration of the pharmaceutical composition to an animal, the biocompatible solvent dissipates in the body of the animal and the biodegradable copolymer forms a biodegradable, non-solid implant in situ in the body of the animal.

17. The pharmaceutical composition of any one of claims 1 to 16 for use as a medicament or in the treatment of a disease.

## Patentansprüche

1. Pharmazeutische Zusammensetzung, umfassend:
a) einen aktiven pharmazeutischen Bestandteil, umfassend ein Peptid oder einen pharmazeutisch akzeptablen Ester oder ein pharmazeutisch akzeptables Salz davon, wobei das Peptid zumindest eine zugängliche Amin-Gruppe umfasst; und
b) ein bioabbaubares flüssiges Block-Copolymer, umfassend:
i) einen Copolymer-Block, umfassend: (1) Monomer-Reste, ausgewählt aus der Gruppe bestehend aus D,L-Laktid, D-Laktid, L-Laktid und Glykolid, und Kombinationen davon, und (2) Monomer-Reste, ausgewählt aus der Gruppe bestehend aus ε-Caprolacton, Trimethylencarbonat und Kombinationen davon; und
ii) einen Polymer-Block, umfassend Polyethylenglykol (PEG) mit niedrigem Molekulargewicht;
wobei das bioabbaubare flüssige Block-Copolymer kein reverses thermisches Gel ist und durch Initiierung mit dem PEG mit niedrigem Molekulargewicht synthetisiert wird; und
wobei eine Viskosität des bioabbaubaren flüssigen Block-Copolymers mit einer Erhöhung der Temperatur nicht spontan zunimmt; und
wobei die Endgruppen des bioabbaubaren flüssigen Block-Copolymers nicht kovalent modifiziert sind.

2. Pharmazeutische Zusammensetzung nach Anspruch 1, wobei das PEG Methoxy-PEG ist.

3. Pharmazeutische Zusammensetzung nach einem der Ansprüche 1 und 2, wobei der Copolymer-Block von (i) ein molares Verhältnis von Laktid- oder Glykolid-Monomer-Resten zu Caprolacton- und/oder Trimethylencarbonat-Monomer-Resten zwischen etwa 10 : 90 und etwa 90 : 10 aufweist.

4. Pharmazeutische Zusammensetzung nach einem der Ansprüche 1 und 2, wobei der Copolymer-Block von (i) ein molares Verhältnis von Laktid- oder Glykolid-Monomer-Resten zu Caprolacton- und/oder Trimethylencarbonat-Monomer-Resten zwischen etwa 25 : 75 und etwa 75 : 25 aufweist.

5. Pharmazeutische Zusammensetzung nach einem der Ansprüche 1 und 2, wobei der Copolymer-Block von (i) ein molares Verhältnis von Laktid-Monomer-Resten zu Caprolacton- und/oder Trimethylencarbonat-Monomer-Resten von 75 : 25 aufweist.

6. Pharmazeutische Zusammensetzung nach einem der Ansprüche 1 bis 5, wobei ein molares Verhältnis von Ethylenglykol-Resten in dem PEG mit niedrigem Molekulargewicht zu allen anderen Monomer-Resten in dem bioabbaubaren Copolymer zwischen etwa 10 : 90 und etwa 50 : 50 beträgt.

7. Pharmazeutische Zusammensetzung nach einem der Ansprüche 1 bis 5, wobei ein molares Verhältnis von Laktid- oder Glykolid-Monomer-Resten zu Caprolacton- und/oder Trimethylencarbonat-Monomeren zu Ethylenglykol-Resten in dem bioabbaubaren Copolymer X:Y:Z beträgt, wobei X eine beliebige Zahl zwischen etwa 25 und etwa 75 sein kann, Y eine beliebige Zahl zwischen etwa 5 und etwa 45 sein kann, und Z eine beliebige Zahl zwischen etwa 5 und etwa 55 sein kann, so dass die Summe von X, Y und Z 100 beträgt.

8. Pharmazeutische Zusammensetzung nach einem der Ansprüche 1 bis 7, wobei das Polyethylenglykol ein zahlenmittleres Molekulargewicht von 200 bis 2000 Dalton aufweist.

9. Pharmazeutische Zusammensetzung nach einem der Ansprüche 1 bis 8, ferner umfassend ein biokompatibles Lösungsmittel oder eine Kombination oder ein Gemisch von Lösemitteln und/oder Co-Lösemitteln.

10. Pharmazeutische Zusammensetzung nach einem der Ansprüche 1 bis 9, ferner umfassend ein zweiwertiges Kation, wobei das zweiwertige Kation als ein Metallsalz bereitgestellt wird oder ausgewählt ist aus der Gruppe bestehend aus Magnesium, Calcium und Zink.

11. Pharmazeutische Zusammensetzung nach Anspruch 10, wobei das Metallsalz ausgewählt ist aus der Gruppe bestehend aus Magnesiumacetat, Magnesiumchlorid, Calciumchlorid, Zinkchlorid und Zinkacetat.

12. Pharmazeutische Zusammensetzung nach Anspruch 10 oder 11, wobei das Metallsalz Magnesiumacetat ist, und wobei das Magnesiumacetat in einer Konzentration von zwischen etwa 0,01 mg/ml und etwa 2,75 mg/ml der pharmazeutischen Zusammensetzung vorliegt; oder
wobei das Metallsalz Magnesiumchlorid ist und wobei das Magnesiumchlorid in einer Konzentration von zwischen etwa 0,01 mg/ml und etwa 3,75 mg/ml der pharmazeutischen Zusammensetzung vorliegt; oder
wobei das Metallsalz Calciumchlorid ist und wobei das Calciumchlorid in einer Konzentration von zwischen etwa 0,01 mg/ml und etwa 1,6 mg/ml der pharmazeutischen Zusammensetzung vorliegt; oder
wobei das Metallsalz Zinkacetat ist und wobei das Zinkacetat in einer Konzentration von zwischen etwa 0,01 mg/ml und etwa 8,2 mg/ml der pharmazeutischen Zusammensetzung vorliegt; oder
wobei das Metallsalz Zinkchlorid ist und wobei das Zinkchlorid in einer Konzentration von zwischen etwa 0,01 mg/ml und etwa 1,4 mg/ml der pharmazeutischen Zusammensetzung vorliegt.

13. Pharmazeutische Zusammensetzung nach einem der Ansprüche 1 bis 12, wobei das Peptid zumindest zwei basische Aminosäuren ausgewählt aus der Gruppe bestehend aus Arginin, Histidin, Lysin und Kombinationen davon umfasst.

14. Pharmazeutische Zusammensetzung nach einem der Ansprüche 1 bis 13, wobei ein gewichtsmittleres Molekulargewicht des bioabbaubaren Copolymers zwischen etwa 1 kDa und etwa 35 kDa beträgt.

15. Pharmazeutische Zusammensetzung nach Anspruch 9, wobei das biokompatible Lösungsmittel N-Methyl-2-Pyrrolidon (NMP) umfasst.

16. Pharmazeutische Zusammensetzung nach Anspruch 9, wobei nach Verabreichung der pharmazeutischen Zusammensetzung an ein Tier das biokompatible Lösungsmittel in den Körper des Tiers dissipiert und das bioabbaubare Copolymer ein bioabbaubares, nicht festes Implantat in situ in dem Körper des Tiers bildet.

17. Pharmazeutische Zusammensetzung nach einem der Ansprüche 1 bis 16 zur Verwendung als ein Medikament oder bei der Behandlung einer Krankheit.

## Revendications

1. Composition pharmaceutique, comprenant :
a) un ingrédient pharmaceutique actif comprenant un peptide, ou un ester ou un sel pharmaceutiquement acceptable de celui-ci, le peptide comprenant au moins un groupe amine accessible ; et
b) un copolymère bloc liquide biodégradable, comprenant :
i) un bloc de copolymère, comprenant (1) des résidus de monomères choisis dans le groupe constitué par le D,L-lactide, le D-lactide, le L-lactide et le glycolide, et des combinaisons de ceux-ci, et (2) des résidus de monomères choisis dans le groupe constitué par l'ε-caprolactone, le carbonate de triméthylène, et des combinaisons de ceux-ci ; et
ii) un bloc de polymère comprenant un polyéthylène glycol (PEG) de faible masse moléculaire ;
le copolymère bloc liquide biodégradable n'étant pas de gel thermique inverse, et étant synthétisé par initiation avec le PEG de faible masse moléculaire ; et
la viscosité du copolymère bloc liquide biodégradable n'augmentant pas spontanément avec une augmentation de la température ; et
les groupes terminaux du copolymère bloc liquide biodégradable n'étant pas modifiés de manière covalente.

2. Composition pharmaceutique selon la revendication 1, dans laquelle le PEG est le méthoxy PEG.

3. Composition pharmaceutique selon l'une quelconque des revendications 1 et 2, dans laquelle le bloc de copolymère de (i) a un rapport molaire des résidus de monomères de lactide ou glycolide et des résidus de monomères de caprolactone et/ou de carbonate de triméthylène entre environ 10 : 90 et environ 90 : 10.

4. Composition pharmaceutique selon l'une quelconque des revendications 1 et 2, dans laquelle le bloc de copolymère de (i) a un rapport molaire des résidus de monomères de lactide ou glycolide et des résidus de monomères de caprolactone et/ou de carbonate de triméthylène entre environ 25 : 75 et environ 75 : 25.

5. Composition pharmaceutique selon l'une quelconque des revendications 1 et 2, dans laquelle le bloc de copolymère de (i) a un rapport molaire des résidus de monomères de lactide et des résidus de monomères de caprolactone et/ou de carbonate de triméthylène de 75 : 25.

6. Composition pharmaceutique selon l'une quelconque des revendications 1 à 5, dans laquelle un rapport molaire des résidus de monomères de l'éthylène glycol dans le PEG de faible masse moléculaire et tous les autres résidus de monomères dans le copolymère biodégradable est entre environ 10 : 90 et environ 50 : 50.

7. Composition pharmaceutique selon l'une quelconque des revendications 1 à 5, dans laquelle un rapport molaire des résidus de monomères de lactide ou glycolide, et des résidus de monomères de caprolactone et/ou de carbonate de triméthylène, et des résidus de l'éthylène glycol dans le copolymère biodégradable est X : Y : Z, où X peut être un nombre quelconque entre environ 25 et environ 75, Y peut être un nombre quelconque entre environ 5 et environ 45, et Z peut être un nombre quelconque entre environ 5 et environ 55, de sorte que la somme de X, Y, et Z soit 100.

8. Composition pharmaceutique selon l'une quelconque des revendications 1 à 7, dans laquelle le polyéthylène glycol a une masse molaire moyenne en nombre de 200 à 2000 dalton.

9. Composition pharmaceutique selon l'une quelconque des revendications 1 à 8, en outre comprenant un solvant biocompatible ou une combination ou un mélange des solvants et/ou co-solvants.

10. Composition pharmaceutique selon l'une quelconque des revendications 1 à 9, en outre comprenant un cation bivalent, le cation bivalent étant fourni en tant que sel de métal ou étant choisi dans le groupe constitué par le magnésium, le calcium et le zinc.

11. Composition pharmaceutique selon la revendication 10, dans laquelle le sel de métal est choisi dans le groupe constitué par l'acétate de magnésium, le chlorure de magnésium, le chlorure de calcium, le chlorure de zinc et l'acétate de zinc.

12. Composition pharmaceutique selon la revendication 10 ou 11, dans laquelle le sel de métal est l'acétate de magnésium, et dans laquelle l'acétate de magnésium est présent à une concentration comprise entre environ 0,01 mg/ml et environ 2,75 mg/ml de la composition pharmaceutique ; ou
dans laquelle le sel de métal est le chlorure de magnésium, et dans laquelle le chlorure de magnésium est présent à une concentration comprise entre environ 0,01 mg/ml et environ 3,75 mg/ml de la composition pharmaceutique ; ou
dans laquelle le sel de métal est le chlorure de calcium, et dans laquelle le chlorure de calcium est présent à une concentration comprise entre environ 0,01 mg/ml et environ 1,6 mg/ml de la composition pharmaceutique ; ou
dans laquelle le sel de métal est l'acétate de zinc, et dans laquelle l'acétate de zinc est présent à une concentration comprise entre environ 0,01 mg/ml et environ 8,2 mg/ml de la composition pharmaceutique ; ou
dans laquelle le sel de métal est le chlorure de zinc, et dans laquelle le chlorure de zinc est présent à une concentration comprise entre environ 0,01 mg/ml et environ 1,4 mg/ml de la composition pharmaceutique.

13. Composition pharmaceutique selon l'une quelconque des revendications 1 à 12, dans laquelle le peptide comprend au moins deux acides aminés basiques choisis dans le groupe constitué par l'arginine, l'histidine, la lysine et leurs combinaisons.

14. Composition pharmaceutique selon l'une quelconque des revendications 1 à 13, dans laquelle la masse moléculaire moyenne en masse du copolymère biodégradable est comprise entre environ 1 kDa et environ 35 kDa.

15. Composition pharmaceutique selon la revendication 9, dans laquelle le solvant biocompatible comprend le N-méthyle-2-pyrrolidone (NMP).

16. Composition pharmaceutique selon la revendication 9, dans laquelle, après administration de la composition pharmaceutique à un animal, le solvant biocompatible se dissipe dans le corps de l'animal et le copolymère biodégradable forme un implant biodégradable et non solide in situ dans le corps de l'animal.

17. Composition pharmaceutique selon l'une quelconque des revendications 1 à 16 pour l'utilisation en tant que médicament ou dans le traitement d'une maladie.
